# EUROPEAN PATENT SPECIFICATION

(11) **EP 3 390 644 B1**
(45) Date of publication and mention of the grant of the patent: **31.07.2024**
(21) Application number: 16822578.7
(22) Date of filing: 14.12.2016
(51) Int. Cl.: C12N 15/86, A01K 67/0276, C12N 9/12, A61K 48/00, A61P 43/00, A61P 31/00, A61P 25/28, A61P 21/00, A61P 11/16, A61P 11/00, A61P 19/00, A61P 1/10, A61P 1/12, A61P 9/00

(54) **ADENO-ASSOCIATED VIRAL VECTORS FOR TREATING MUCOLIPIDOSIS TYPE II**
ADENO-ASSOZIIERTE VIRALE VEKTOREN ZUR BEHANDLUNG VON MUCOLIPIDOSE TYP II
VECTEURS VIRAUX ADÉNO-ASSOCIÉS POUR TRAITER LA MUCOLIPIDOSE DE TYPE II

(30) Priority: 15.12.2015 US 201562267502 P
(43) Date of publication of application: 24.10.2018
(73) Proprietor: Genzyme Corporation, Cambridge, MA 02142 (US); Samsung Life Public Welfare Foundation, Seoul 04348 (KR)
(72) Inventor: YEW, Nelson, Bridgewater, NJ 08807 (US); JIN, Dong-Kyu, Seoul (KR)
(74) Representative: Lavoix
(86) International application number: PCT/US2016/066611
(87) International publication number: WO 2017/106313

(56) References cited:
- WO-A1-2008/154198
- WO-A2-2015/168666
- DATABASE UniProt [online] 7 March 2006 (2006-03-07), "RecName: Full=N-acetylglucosamine-1-phosphotransferase subunits alpha/beta; EC=2.7.8.17 {ECO:0000269|PubMed:19955174}; AltName: Full=GlcNAc-1-phosphotransferase subunits alpha/beta; AltName: Full=Stealth protein GNPTAB; AltName: Full=UDP-N-acetylglucosamine-1-phosphotransferase subunits alpha/beta;", XP002766821, retrieved from EBI accession no. UNIPROT:Q3T906 Database accession no. Q3T906
- STEVEN J. GRAY ET AL: "Optimizing Promoters for Recombinant Adeno-Associated Virus-Mediated Gene Expression in the Peripheral and Central Nervous System Using Self-Complementary Vectors", HUMAN GENE THERAPY, vol. 22, no. 9, September 2011 (2011-09-01), pages 1143 - 1153, XP055198141, ISSN: 1043-0342, DOI: 10.1089/hum.2010.245
- ARONOVICH ELENA L ET AL: "Lysosomal storage disease: Gene therapy on both sides of the blood-brain bar", MOLECULAR GENETICS AND METABOLISM, vol. 114, no. 2, 7 October 2014 (2014-10-07), pages 83 - 93, XP029223949, ISSN: 1096-7192, DOI: 10.1016/J.YMGME.2014.09.011
- P. VOGEL ET AL: "Comparative Pathology of Murine Mucolipidosis Types II and IIIC", VETERINARY PATHOLOGY., vol. 46, no. 2, March 2009 (2009-03-01), US, pages 313 - 324, XP055342280, ISSN: 0300-9858, DOI: 10.1354/vp.46-2-313
- L. PATON ET AL: "A Novel Mouse Model of a Patient Mucolipidosis II Mutation Recapitulates Disease Pathology", JOURNAL OF BIOLOGICAL CHEMISTRY, vol. 289, no. 39, 26 September 2014 (2014-09-26), US, pages 26709 - 26721, XP055342424, ISSN: 0021-9258, DOI: 10.1074/jbc.M114.586156
- KO AH-RA ET AL: "AAV8-mediated expression of N-acetylglucosamine-1-phosphate transferase attenuates bone loss in a mouse model of mucolipidosis II", MOLECULAR GENETICS AND METABOLISM, ACADEMIC PRESS, AMSTERDAM, NL, vol. 117, no. 4, 3 February 2016 (2016-02-03), pages 447 - 455, XP029486946, ISSN: 1096-7192, DOI: 10.1016/J.YMGME.2016.02.001

## Description

### CROSS REFERENCE TO RELATED APPLICATIONS

This application claims the priority benefit of U.S. Provisional Application Serial No. 62/267,502, filed December 15, 2015.

### FIELD OF THE INVENTION

The present invention relates to rAAV vectors, particles, compositions, for use for treating mucolipidosis type II and/or type III.

### BRIEF SUMMARY OF THE INVENTION

Mucolipidosis types II and III (ML II; ML III) are autosomal recessive lysosomal storage diseases characterized by a deficiency of UDP-GlcNAc;Lysosomal enzyme N-acetylglucosamine-1-phosphotransferase (abbreviated GlcNAc-1-phosphotransferase). GlcNAc-1-phosphotransferase is a hexameric enzyme complex consisting of three different subunits: α₂, β₂, and γ₂. The α₂, and β₂ subunits contain the catalytic activity and are encoded by a single gene, GNPTAB. Mutations in GNPTAB that result in a complete loss of enzymatic activity are found in patients with MLII. Mucolipidosis II is highly progressive, and patients rarely survive past the first decade of life. In addition, the available treatment options for ML II remain limited. Only bone marrow transplantation has been attempted in a small number of patients. In contrast, mutations in GNPTAB resulting in a partial reduction in GlcNAc-1-phosphotransferase activity are found in patients with MLIII. These patients generally show less severe symptoms and slower disease progression but still present with serious health problems such as skeletal abnormalities, developmental delay, and cardiomegaly.

The availability of AAV serotypes that display tissue-specific tropism and promote sustained expression of transgenes offers the possibility of AAV-mediated gene therapy for the systemic treatment of lysosomal disease, including ML II and ML III. Accordingly, investigation of AAV-mediated treatment of ML II/III is important to establish whether this approach represents a potential therapeutic strategy for this devastating disease. However, before AAV-mediated treatment of MLII/III can be examined, technical limitations need to be overcome. The coding sequence of GNPTAB (over 5.6 kb in humans) is longer than the endogenous AAV genome (approximately 4.7 kb). Therefore, AAV vectors that can accommodate GNPTAB along with functional promoter/enhancer sequences and the other components of the AAV genome while still facilitating efficient packaging into viral particles are highly advantageous.

The invention is as defined by the claims. The invention relates to a recombinant adeno-associated virus (rAAV) particle for use in a method for treating mucolipidosis type II (ML II) or mucolipidosis type III (ML III) in a mammal,
wherein the rAAV particle comprises a rAAV vector, wherein the rAAV vector comprises nucleic acid encoding N-acetylglucosamine-1-phosphate transferase (GNPTAB), at least one AAV inverted terminal repeat (ITR), and a minute virus of mice (MVM) intron,
wherein the GNPTAB is operably linked to a promoter, wherein the promoter is a cytomegalovirus (CMV) enhancer/chicken beta-actin (CBA) promoter, wherein the CMV enhancer is a shortened enhancer and the CBA promoter is a truncated CBA promoter.

The application discloses recombinant adeno-associated virus (rAAV) vector comprising nucleic acid encoding N-acetylglucosamine-1-phosphate transferase (GNPTAB) and at least one AAV inverted terminal repeat (ITR). In some embodiments, the GNPTAB comprises the alpha and beta subunits. According to the invention, the GNPTAB is operably linked to a promoter. In some embodiments, the GNPTAB is a human GNPTAB. In some embodiments, the GNPTAB comprises an amino acid sequence that is at least about 80%, at least about 85%, at least about 90% or at least about 95% identical to the amino acid sequence of SEQ ID NO:1. In some embodiments, the GNPTAB comprises the amino acid sequence of SEQ ID NO: 1. According to the invention, the promoter is a modified CBA promoter which is a truncated CBA promoter. According to the invention, the CMV enhancer is a shortened CMV enhancer. According to the invention, the vector comprises an MVM intron. In some embodiments, the vector comprises a polyadenylation sequence. In some embodiments, the polyadenylation sequence is a bovine growth hormone polyadenylation sequence. In some embodiments, the AAV terminal repeat is an AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV DJ, a goat AAV, bovine AAV, or mouse AAV serotype ITR. In some embodiments, the rAAV vector comprises two ITRs.

In some aspects, the application discloses a rAAV particle comprising the rAAV vector of any one of the above embodiments. In some embodiments, the AAV particle comprises an AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV2/2-7m8, AAV DJ, AAV2 N587A, AAV2 E548A, AAV2 N708A, AAV V708K, goat AAV, AAV1/AAV2 chimeric, bovine AAV, mouse AAV, or rAAV2/HBoV1 serotype capsid. In some embodiments, the rAAV particle comprises one or more ITRs and capsid derived from the same AAV serotype. In some embodiments, the rAAV particle comprises one or more ITRs derived from a different AAV serotype than capsid of the rAAV viral particles. In some embodiments, the rAAV particle comprises an AAV8 capsid, and wherein the vector comprises AAV2 ITRs. In some embodiments, the rAAV particle is produced by transfecting a host cell with nucleic acid encoding the rAAV vector and nucleic acid encoding AAV rep and cap functions, and providing nucleic acid encoding AAV helper functions. In some embodiments, the AAV helper functions are provided by transfecting the host cell with nucleic acid encoding the AAV helper functions. In some embodiments, the AAV helper functions are provided by infecting the host cell with an AAV helper virus that provides the AAV helper functions. In some embodiments, the AAV helper virus is an adenovirus, a herpes simplex virus or a baculovirus. In some embodiments, the rAAV particle is produced by an AAV producer cell comprising nucleic acid encoding the rAAV vector and nucleic acid encoding AAV rep and cap functions, and providing nucleic acid encoding AAV helper functions. In some embodiments, the AAV producer cell comprises nucleic acid encoding AAV helper functions. In some embodiments, the AAV helper functions are provided by infecting the AAV producer cells with an AAV helper virus that provides the AAV helper functions. In some embodiments, the AAV helper virus is an adenovirus, a herpes simplex virus, or a baculovirus. In some embodiments, the invention provides a pharmaceutical composition comprising any of the rAAV particles described herein.

The invention provides the rAAV particle for use in a method for treating mucolipidosis type II (ML II) or mucolipidosis type III (ML III) in a mammal as defined in the claims. In some embodiments, the method is for maintaining or increasing body size in a mammal with mucolipidosis type II (ML II) or mucolipidosis type III (ML III), wherein the rAAV particles comprise a rAAV vector, wherein the rAAV vector comprises nucleic acid encoding N-acetylglucosamine-1-phosphate transferase (GNPTAB) and at least one AAV ITR, wherein expression of the GNPTAB results in maintenance of or an increase in body weight gain and/or maintenance of or an increase in body height gain. In some embodiments, the method is for preventing the reduction of body size in a mammal with mucolipidosis type II (ML II) or mucolipidosis type III (ML III) wherein the rAAV particles comprise a rAAV vector, wherein the rAAV vector comprises nucleic acid encoding N-acetylglucosamine-1-phosphate transferase (GNPTAB) and at least one AAV ITR, wherein expression of the GNPTAB prevents the reduction of body weight. In some embodiments, the method is for maintaining or increasing bone mineral content in a mammal with mucolipidosis type II (ML II) or mucolipidosis type III (ML III) wherein the rAAV particles comprise a rAAV vector, wherein the rAAV vector comprises nucleic acid encoding GNPTAB and at least one AAV ITR, wherein expression of the GNPTAB results in maintenance or an increase in bone mineral content. In some embodiments, the method is for preventing the reduction of bone mineral content in a mammal with mucolipidosis type II (ML II) or mucolipidosis type III (ML III) wherein the rAAV particles comprise a rAAV vector, wherein the rAAV vector comprises nucleic acid encoding GNPTAB and at least one AAV ITR, wherein expression of the GNPTAB prevents reduction in bone mineral content. In some embodiments, the method is for maintaining or increasing bone mineral density in a mammal with mucolipidosis type II (ML II) or mucolipidosis type III (ML III) wherein the rAAV particles comprise a rAAV vector, wherein the rAAV vector comprises nucleic acid encoding GNPTAB and at least one AAV ITR, wherein expression of the GNPTAB results in maintenance or an increase in bone mineral density. In some embodiments, the method is for preventing the reduction of bone mineral density in a mammal with mucolipidosis type II (ML II) or mucolipidosis type III (ML III) wherein the rAAV particles comprise a rAAV vector, wherein the rAAV vector comprises nucleic acid encoding GNPTAB and at least one AAV ITR, wherein expression of the GNPTAB prevents reduction in bone mineral density.

In some embodiments of the above uses, the treatment ameliorates one or more symptoms of ML II or ML III, wherein the one or more symptoms of ML II or ML III are skeletal defects, cognitive deficits, delays in the development of gross and fine motor skills, hearing loss, lack of muscle tone, protruded abdomen, umbilical hernias, progressive mucosal thickening of the airways, frequent respiratory infections, thickening and insufficiency of the mitral valve, constipation or diarrhea. In some embodiments, the treatment delays to progression of one or more symptoms of ML II or ML III, wherein the one or more symptoms of ML II or ML III are skeletal defects, cognitive deficits, delays in the development of gross and fine motor skills, hearing loss, lack of muscle tone, protruded abdomen, umbilical hernias, progressive mucosal thickening of the airways, frequent respiratory infections, thickening and insufficiency of the mitral valve, constipation or diarrhea. In some embodiments, method is for ameliorating one or more symptoms of ML II or ML III in a mammal wherein the rAAV particles comprise a rAAV vector, wherein the rAAV vector comprises nucleic acid encoding GNPTAB and at least one AAV ITR; wherein the one or more symptoms of ML II or ML III are skeletal defects, cognitive deficits, delays in the development of gross and fine motor skills, hearing loss, lack of muscle tone, protruded abdomen, umbilical hernias, progressive mucosal thickening of the airways, frequent respiratory infections, thickening and insufficiency of the mitral valve, constipation or diarrhea. In some embodiments, the method is for delaying the progression of one or more symptoms of ML II or ML III in a mammal wherein the rAAV particles comprise a rAAV vector, wherein the rAAV vector comprises nucleic acid encoding GNPTAB and at least one AAV ITR; wherein the one or more symptoms of ML II or ML III are skeletal defects, cognitive deficits, delays in the development of gross and fine motor skills, hearing loss, lack of muscle tone, protruded abdomen, umbilical hernias, progressive mucosal thickening of the airways, frequent respiratory infections, thickening and insufficiency of the mitral valve, constipation or diarrhea.

According to the invention, the GNPTAB is operably linked to a promoter. In some embodiments, the GNPTAB is a human GNPTAB. In some embodiments, the GNPTAB comprises an amino acid sequence that is at least about 80% identical to the amino acid sequence of SEQ ID NO:1. In some embodiments, the GNPTAB comprises the amino acid sequence of SEQ ID NO:1. According to the invention, the promoter is a CMV enhancer/chicken beta-actin (CBA) promoter. According to the invention, the CBA promoter is a modified CBA promoter. According to the invention, the modified CBA promoter is a truncated CBA promoter. According to the invention, the CMV enhancer is a shortened CMV enhancer. According to the invention, the vector comprises an MVM intron. In some embodiments, the vector comprises a polyadenylation sequence. In some embodiments, the polyadenylation sequence is a bovine growth hormone polyadenylation sequence. In some embodiments, the AAV terminal repeat is an AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV DJ, a goat AAV, bovine AAV, or mouse AAV serotype ITR. In some embodiments, the rAAV vector comprises two ITRs. In some embodiments, the AAV particle comprises an AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV2/2-7m8, AAV DJ, AAV2 N587A, AAV2 E548A, AAV2 N708A, AAV V708K, goat AAV, AAV1/AAV2 chimeric, bovine AAV, mouse AAV, or rAAV2/HBoV1 serotype capsid. In some embodiments, the rAAV particle comprises one or more ITRs and capsid derived from the same AAV serotype. In some embodiments, the rAAV particle comprises one or more ITRs derived from a different AAV serotype than capsid of the rAAV viral particles. In some embodiments, the rAAV particle comprises an AAV8 capsid, and wherein the vector comprises AAV2 ITRs.

In some embodiments of the above embodiments, the rAAV particle is produced by transfecting a host cell with nucleic acid encoding the rAAV vector and nucleic acid encoding AAV rep and cap functions, and providing nucleic acid encoding AAV helper functions. In some embodiments, the AAV helper functions are provided by transfecting the host cell with nucleic acid encoding the AAV helper functions. In some embodiments, the AAV helper functions are provided by infecting the host cell with an AAV helper virus that provides the AAV helper functions. In some embodiments, the AAV helper virus is an adenovirus, a herpes simplex virus, or a baculovirus. In some embodiments, the rAAV particle is produced by an AAV producer cell comprising nucleic acid encoding the rAAV vector and nucleic acid encoding AAV rep and cap functions, and providing nucleic acid encoding AAV helper functions. In some embodiments, the AAV producer cell comprises nucleic acid encoding AAV helper functions. In some embodiments, the AAV helper functions are provided by infecting the AAV producer cells with an AAV helper virus that provides the AAV helper functions. In some embodiments, the AAV helper virus is an adenovirus, a herpes simplex virus, or a baculovirus.

In some embodiments of the above uses, the mammal is a human. In some embodiments, the human is a pediatric subject. In some embodiments, the human is a young adult.

In some embodiments of the above uses, the rAAV is administered intravenously, intraperitoneally, intra-arterially, intramuscularly, subcutaneously, or intrahepatically. In some embodiments, the rAAV is administered intravenously. In some embodiments, the rAAV is administered to more than one location. In some embodiments, the administration is repeated. In some embodiments, the rAAV viral particles are in a pharmaceutical composition. In some embodiments, the pharmaceutical composition further comprises a pharmaceutically acceptable carrier.

In some aspects, the application discloses the use of any pharmaceutical composition as described herein the manufacture of a medicament for treating ML II or ML III in a mammal. In some aspects, the application discloses the use of any pharmaceutical composition as described herein in the manufacture of a medicament for use in any of the methods described herein. In some aspects, the application discloses the use of any of the rAAV particles as described herein in the manufacture of a medicament for treating ML II or ML III in a mammal. In some aspects, the application discloses the use of any of the rAAV particles described herein the manufacture of a medicament for use in any of the methods as described herein. The invention provides any of the pharmaceutical compositions described herein for use for treating ML II or ML III in a mammal. In some aspects, the application discloses the use of any of the pharmaceutical compositions described herein for use in any of the methods described herein. The application discloses the use of any of the recombinant AAV described herein for treating ML II or ML III in a mammal. In some aspects, the application discloses the use of any of the recombinant AAV described herein for use in any of the methods described herein. In some aspects, the application discloses the use any of the pharmaceutical compositions described herein in the manufacture of a medicament for ameliorating one or more symptoms of ML II or ML III in a mammal or delaying the progression of one or more symptoms of ML II or ML III in a mammal. In some embodiments, the invention provides any rAAV particles described herein for use for ameliorating one or more symptoms of ML II or ML III in a mammal or delaying the progression of one or more symptoms of ML II or ML III in a mammal. In some embodiments, the invention provides any pharmaceutical composition described herein for use for ameliorating one or more symptoms of ML II or ML III in a mammal or delaying the progression of one or more symptoms of ML II or ML III in a mammal. In some aspects, the the application discloses the use of any recombinant AAV described herein for ameliorating one or more symptoms of ML II or ML III in a mammal or delaying the progression of one or more symptoms of ML II or ML III in a mammal. In some embodiments, the one or more symptoms of ML II or ML III are skeletal defects, cognitive deficits, delays in the development of gross and fine motor skills, hearing loss, lack of muscle tone, protruded abdomen, umbilical hernias, progressive mucosal thickening of the airways, frequent respiratory infections, thickening and insufficiency of the mitral valve, constipation or diarrhea. In some embodiments, the mammal is a human.

In some aspects, the application discloses a kit comprising any of the rAAV vectors described herein, any of the rAAV particles described herein or any pharmaceutical composition as described herein. In some aspects, the kit is for treating ML II or ML III according any of the methods as described herein. In some aspects, the kit comprises any of the rAAV vectors as described herein, any of the rAAV particles as described herein or any pharmaceutical composition as described herein. In some aspects, the kit further comprises one or more buffers or pharmaceutically acceptable excipients. In some aspects, the kit further comprises instructions for use in treating ML II and/or ML III.

Not part of the invention, the application discloses a non-human animal model of Mucoliposis II (ML II) wherein at least one allele of a N-acetylglucosamine-1-phosphate transferase (GNPTAB) gene comprises a deletion located between exons 12 and exon 20. In some aspects, at least one allele of the GNPTAB gene comprises a deletion spanning exons 12 and exon 20. In some aspects, the animal is homozygous for the deletion in the GNPTAB gene. In some aspects, the animal is heterozygous for the deletion in the GNPTAB gene. In some aspects, a portion of the GNPTAB gene is replaced by a gene encoding a reporter and/or selectable marker. In some aspects, the selectable marker confers resistance to neomycin. In some aspects, the animal is a mammal. In some aspects, the mammal is a rodent. In some embodim aspects ents, the rodent is a mouse. In some aspects, the mouse has a genetic background derived from 129/Sv and/or C57Bl/6. In some aspects, the animal is immunocompetent or immunodeficient.

Not part of the invention, the application discloses a method of generating a non-human animal model of Mucolipidosis II (ML II) comprising introducing a deletion between exons 12 and 20 in at least one allele of the GNPTAB gene on the animal. In some aspects, at least one allele of the GNPTAB gene comprises a deletion spanning exons 12 and exon 20. In some aspects, the animal is bred to be homozygous for the deletion in the GNPTAB gene. In some aspects, the animal is bred to be heterozygous for the deletion in the GNPTAB gene. In some aspects, a portion of the GNPTAB gene is replaced by a gene encoding a reporter and/or selectable marker. In some aspects, the selectable marker confers resistance to neomycin. In some embodiments, the animal is a mammal. In some aspects, the mammal is a rodent. In some aspects, the rodent is a mouse. In some aspects, the mouse has a genetic background derived from 129/Sv and/or C57Bl/6. In some aspects, the animal is immunocompetent or immunodeficient.

Not part of the invention, the application discloses a method for evaluating an agent for treatment of Mucolipidosis II (ML II) comprising administering the agent to the non-human animal model as described herein, wherein amelioration one or more symptoms of ML II indicates the agent may provide beneficial treatment of ML II. In some aspects, the symptom of ML II is decreased body weight, decreased bone density, decreased bone mineral content, skeletal defects, cognitive deficits, delays in the development of gross and fine motor skills, hearing loss, lack of muscle tone, protruded abdomen, umbilical hernias, progressive mucosal thickening of the airways, frequent respiratory infections, thickening and insufficiency of the mitral valve, constipation and/or diarrhea. In some aspects, the agent is a small molecule, a polypeptide, an antibody, a nucleic acid or a recombinant viral particle.

### BRIEF DESCRIPTION OF THE DRAWINGS

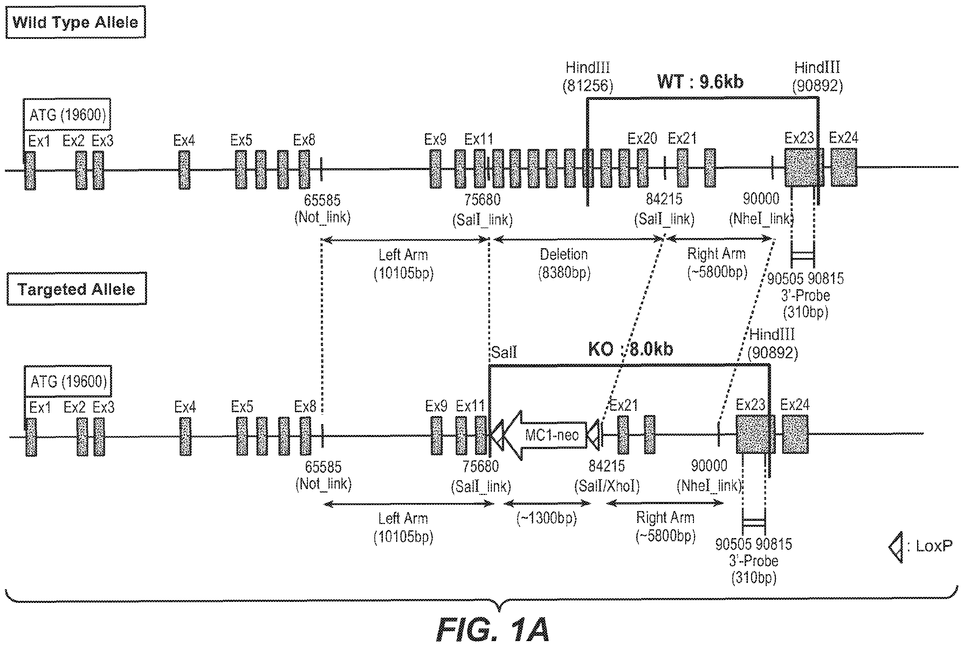

**FIG. 1A** provides a diagram of the mouse GNPTAB gene structure and the genomic insertion site of the gene trapping vector used for generating GNPTAB knockout mice.

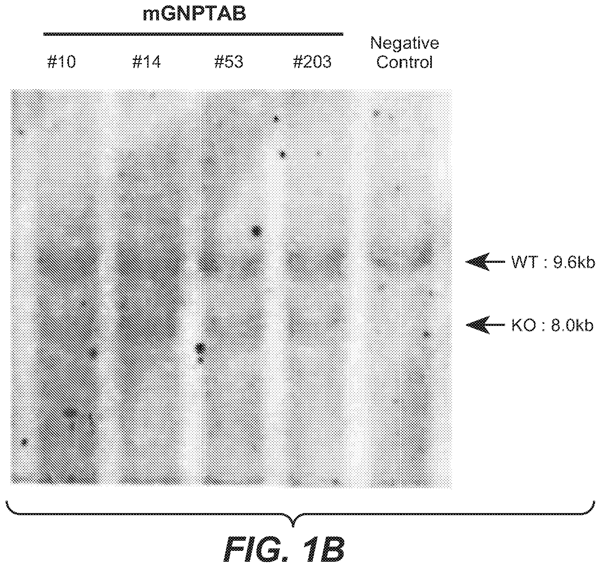

**FIG. 1B** is a Southern blot showing mouse ES clones used for generating GNPTAB knockout mice.

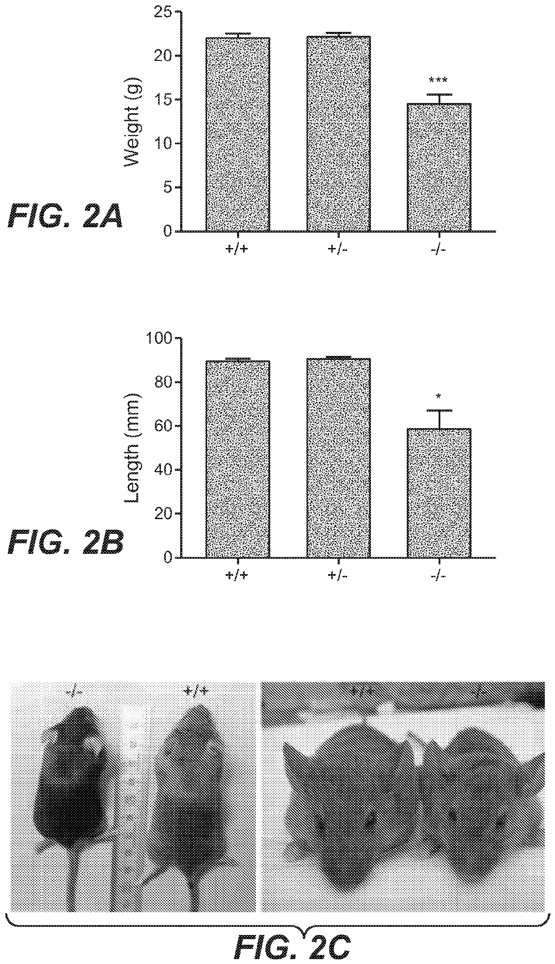

**FIGS. 2A-2C** show the growth retardation present in GNPTAB knock-out mice. (**FIG. 2A**) Body weight (g) of wild type (+/+), heterozygous (+/-), and homozygous (-/-) mice at 6 weeks of age (***; *p<0.0001* ; Bonterroni multiple comparison test). (**FIG. 2B**) Naso-anal length (mm) of wild type, heterozygous, and homozygous mice at 6 weeks of (*; *p<0.02 ;* Bonterroni multiple comparison test). (**FIG. 2C**) Gross morphology of wild type and homozygous mice.

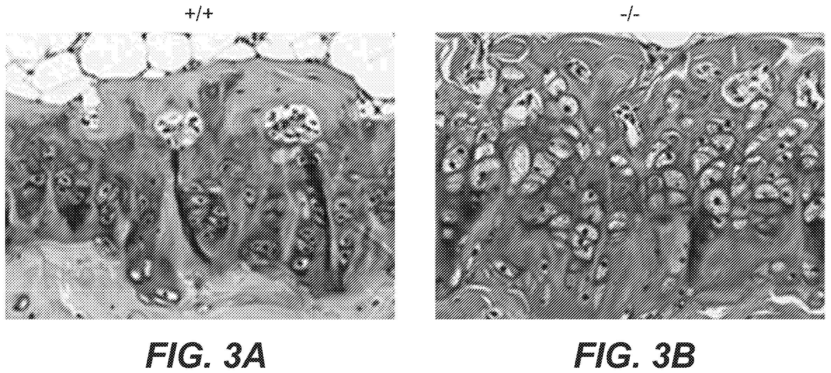

**FIGS. 3A & 3B** show light microscopic images of representative sections of hematoxylin and eosin-stained femoral cartilage from wild type (**FIG. 3A**) and homozygous knockout (**FIG. 3B**) mice.

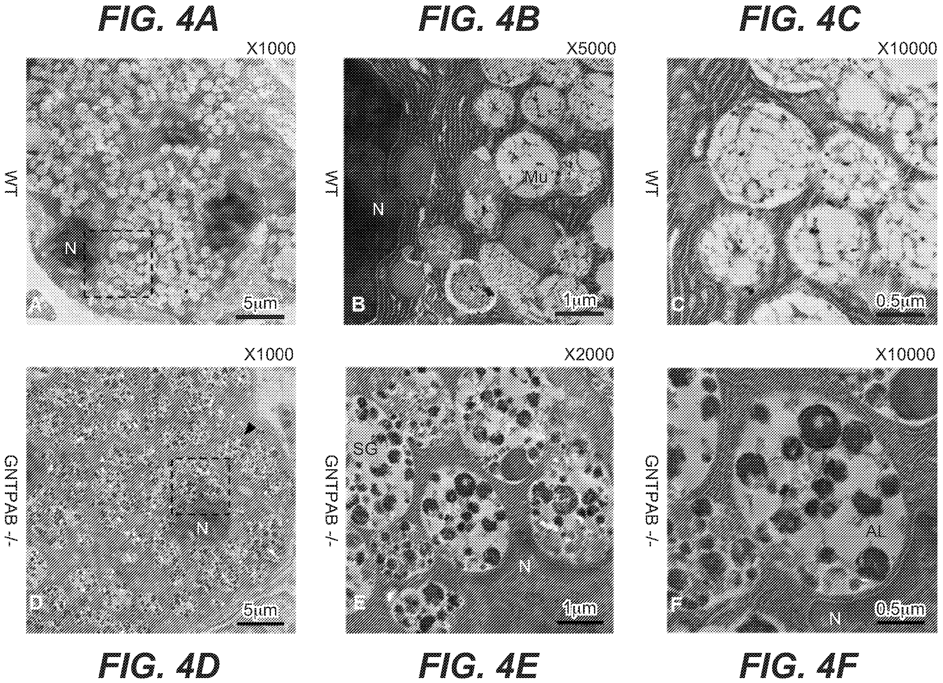

**FIGS. 4A-4F** demonstrates the accumulation of autolysosomes in the salivary glands of knockout (KO) mice. (**FIGS. 4A-C**) EM showing an overview of wild type mouse salivary gland acinus, composed of mucous and serous cells. (**FIG. 4D**) Overview of a KO salivary gland acinus. The overall architecture is disrupted by the massive accumulation of large vacuoles in KO. (**FIGS. 4E-F**) Higher magnification of a vacuole from (**FIG. 4D**), which is surrounded by a single membrane and contains un-degraded material. Area of magnification is indicated by the box in (**FIG. 4D**). AL, autolysosome; Mu, mucous cell; N, nucleus; SG; secretory granule.

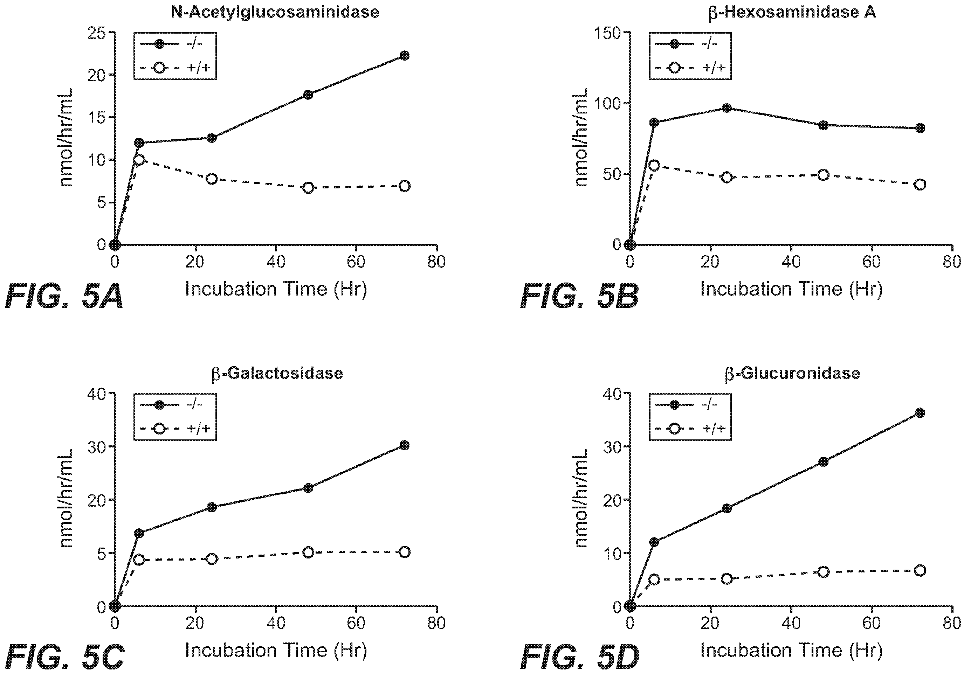

**FIGS. 5A-5D** show lysosomal enzyme activities in sera of wild type (open circles) and KO mice (filled circles), as labeled. Activity of N-acetylglucosaminidase (**FIG. 5A**), β-hexosaminidase A (**FIG. 5B**), β-galactosidase (**FIG. 5C**), and β-glucuronidase (**FIG. 5D**) are shown.

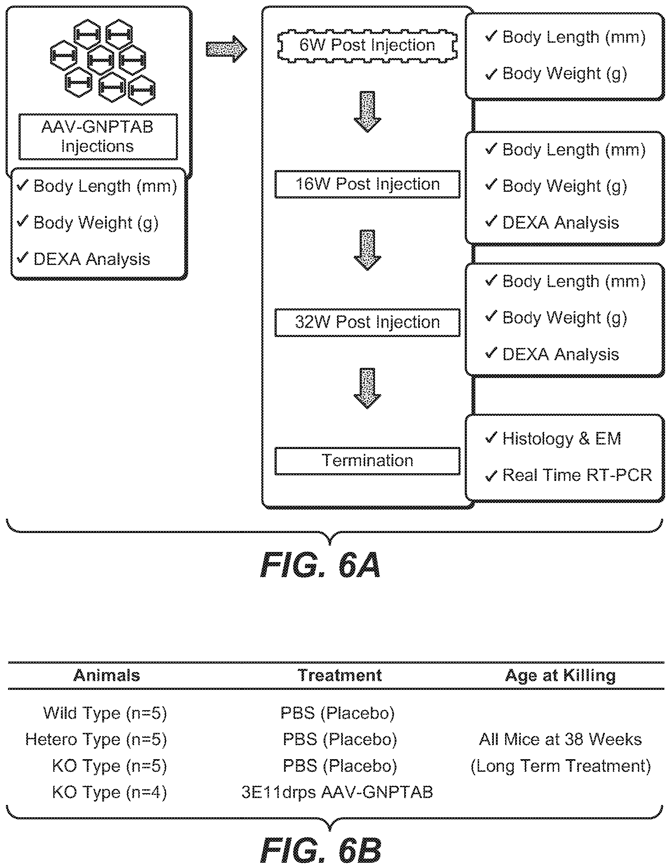

**FIGS. 6A & 6B** provide an overview of the experimental timeline for injections. (**FIG. 6A**) Timelines for long term treatment of the study for mice injected with viral vector in 6 weeks-old age. (**FIG. 6B**) The total number of mice (n) injected for each treatment group is indicated.

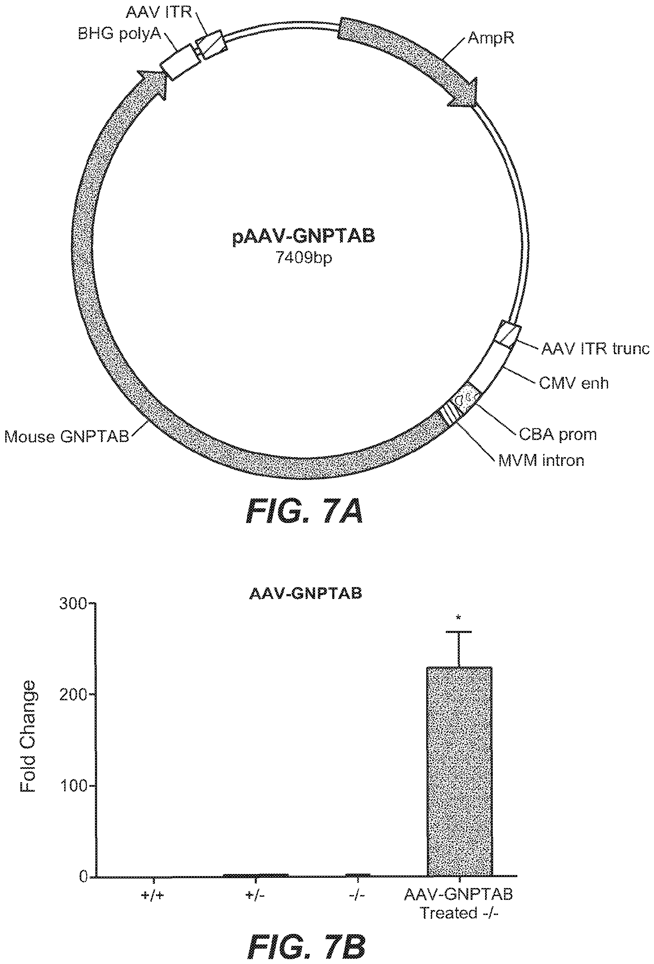

**FIG. 7A** depicts a schematic of the pAAV2/8-GNPTAB vector containing mouse GNPTAB cDNA. The mouse GNPTAB cDNA sequence is based on GenBank accession number NM_001004164.2. The nucleotide sequence was codon-optimized for expression in mouse. The amino acid sequence is unchanged.
**FIG. 7B** shows the quantitative analysis of livers from KO mice injected with AAV-GNPTAB as compared to their control littermates.

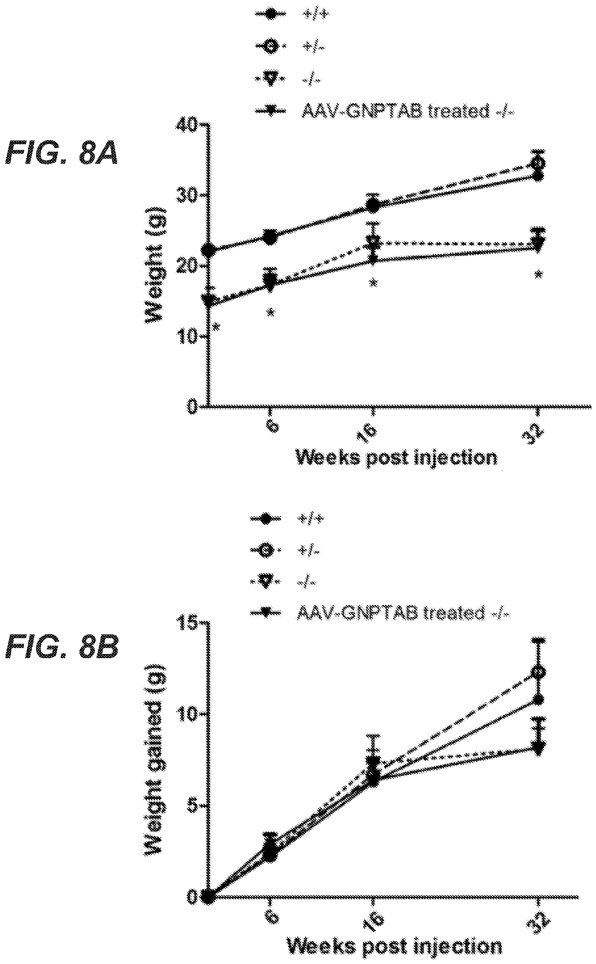

**FIGS. 8A & 8B** show the body weight change from starting weight over time for control and AAV-GNPTAB treated KO mice. (**FIG. 8A**) Total body weight of control mice and AAV- GNPTAB treated KO mice (**p*< 0.05 Dunnett multiple comparison test). (**FIG. 8B**) Data expressed as amount of weight change from starting weight over time.

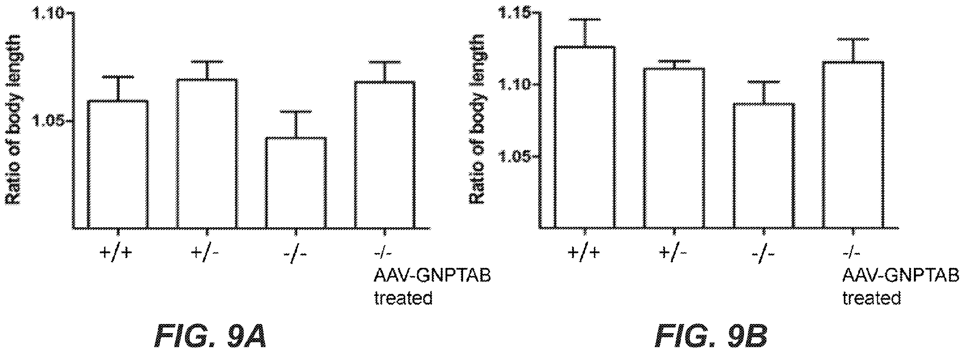

**FIGS. 9A & 9B** show the height change from starting height over time for control and AAV-GNPTAB treated KO mice. (**FIG. 9A**) Data expressed as the ratio of body length compared before and 6 weeks post injection (**FIG. 9B**) Data expressed as the ratio of body length compared before and 32 weeks post injection.

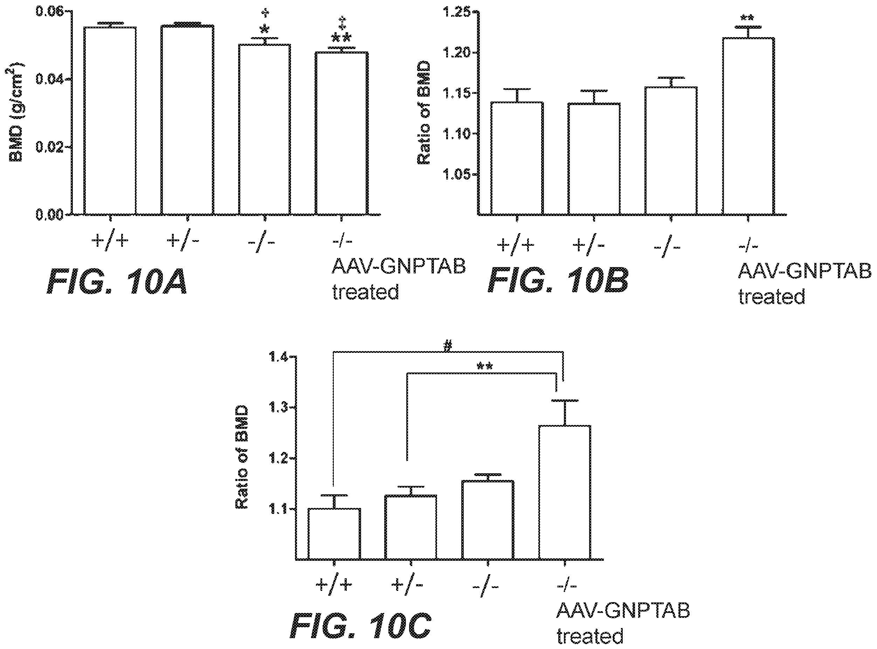

**FIGS. 10A-10C** provide histograms showing levels of bone mineral density before injection (**FIG. 10A**), at 16 weeks post injection (**FIG. 10B**), and at 32 weeks post injection (**FIG. 10C**). (**FIG. 10A**) The data from homozygous and homozygous treated with AAV-GNPTAB were compared with that of wild type and heterozygous. (†; *p<0.05*, ‡; *p<0.02* compared with wild type, *; *p<0.02*, **; *p<0.002* compared with heterozygous). AAV-GNPTAB treatment resulted in a statistically significant increase in BMD ratio (post/before Tx) in homozygous mice post16 weeks treatment (**FIG. 10B**) and 32 weeks treatment (**FIG**. **10C**). (**FIG. 10B**) 16 weeks after treatment, GNPTAB null mice treated with AAV-GNPTAB showed a significantly increase in BMD ratio than other mice (**; *P<0.02*) (**FIG. 10C**) 32 weeks after treatment, significant differences in BMD ratio was observed in GNPTAB treated mice. (#; *p<0.05*, **; *p<0.02*)*. P* values were determined by two-tailed, unpaired t-test analyses. Data are presented as Mean ± SEM.

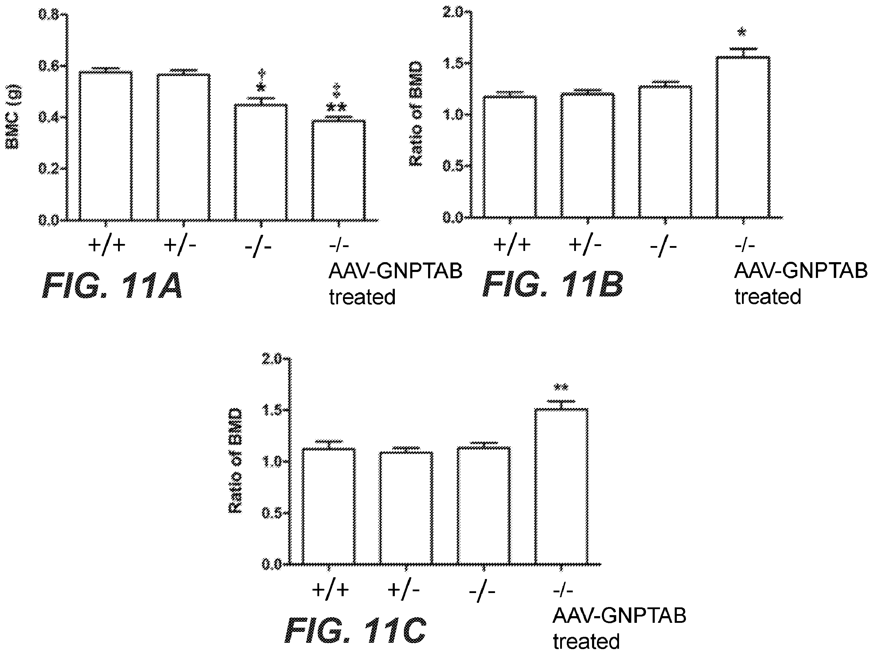

**FIGS. 11A-11C** provide histograms provide histograms showing levels of bone mineral content before injection (**FIG. 11A**), at 16 weeks post injection (**FIG. 11B**), and at 32 weeks post injection (**FIG. 11C**). (**FIG. 11A**) The data from homozygous, and homozygous treated with AAV-GNPTAB were compared with that of wild type and heterozygous. (†; *p<0.05*, ‡; *p<0.02* compared with wild type, *; *p<0.02*, **; *p<0.002* compared with heterozygous). AAV-GNPTAB treatment resulted in a statistically significant increase in BMD ratio (post/before Tx) in homozygous mice post16 weeks treatment (**FIG. 11B**) and 32 weeks treatment (**FIG. 11C**). (**FIG. 11B**) 16 weeks after treatment, GNPTAB null mice treated with AAV-GNPTAB showed a significantly increase in BMD ratio than other mice (**; *P<0.02*) (**FIG. 11C**) 32 weeks after treatment, significant differences in BMD ratio was observed in GNPTAB treated mice. (#; *p<0.05*, **; *p<0.02*)*. P* values were determined by two-tailed, unpaired t-test analyses. Data are presented as Mean ± SEM.

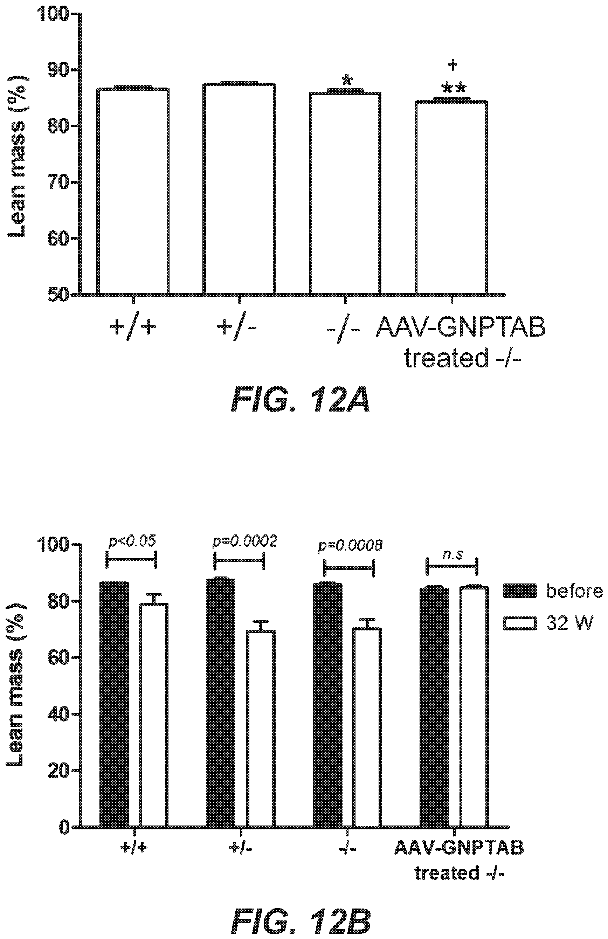

**FIGS. 12A & 12B** provide histograms showing levels of percent lean mass before injection (**FIG. 12A**) and change of percent lean mass 32 weeks post injection (**FIG. 12B**). (**FIG. 12A**) The data from homozygous, and homozygous treated with AAV-GNPTAB were compared with that of wild type and heterozygous. (†; *p*<0.02 compared with wild type, *; *p*<0.05, **; *p*<0.001 compared with heterozygous). (**FIG. 12B**) 32 weeks after treatment, no change in % lean mass was observed in AAV-GNPTAB treated mice. *P* values were determined by two-tailed, unpaired t-test analyses. Data are presented as Mean ± SEM.

### DETAILED DESCRIPTION

In some aspects, the application discloses a recombinant adeno-associated virus (rAAV) vector comprising nucleic acid encoding N-acetylglucosamine-1-phosphate transferase, alpha and beta subunits (GNPTAB) and at least one AAV inverted terminal repeat (ITR). Further disclosed herein are rAAV particles comprising a rAAV vector of the present disclosure, as well as pharmaceutical compositions comprising a rAAV particle of the present disclosure.

In some aspects, the application further methods for treating mucolipidosis type II (ML II) or mucolipidosis type III (ML III) in a mammal comprising administering to the mammal an effective amount of rAAV particles, where the rAAV particles comprise a rAAV vector, and the rAAV vector comprises nucleic acid encoding GNPTAB and at least one AAV ITR. Further provided herein are methods for increasing body size, bone mineral content, and/or bone mineral density in a mammal with mucolipidosis type II (ML II) or mucolipidosis type III (ML III) comprising administering to the mammal an effective amount of rAAV particles, where the rAAV particles comprise a rAAV vector, and the rAAV vector comprises nucleic acid encoding GNPTAB and at least one AAV ITR. In some embodiments, the expression of the GNPTAB results in an increase in body size, bone mineral content, and/or bone mineral density.

In some aspects, the application further discloses uses and/or kits for treating ML II or ML III, *e.g.*, using a rAAV vector, rAAV particle, or pharmaceutical composition of the present disclosure.

### 1. General Techniques

The techniques and procedures described or referenced herein are generally well understood and commonly employed using conventional methodology by those skilled in the art, such as, for example, the widely utilized methodologies described in Molecular Cloning: A Laboratory Manual (Sambrook et al., 4th ed., Cold Spring Harbor Laboratory Press, Cold Spring Harbor, N.Y., 2012); Current Protocols in Molecular Biology (F.M. Ausubel, et al. eds., 2003); the series Methods in Enzymology (Academic Press, Inc.); PCR 2: A Practical Approach (M.J. MacPherson, B.D. Hames and G.R. Taylor eds., 1995); Antibodies, A Laboratory Manual (Harlow and Lane, eds., 1988); Culture of Animal Cells: A Manual of Basic Technique and Specialized Applications (R.I. Freshney, 6th ed., J. Wiley and Sons, 2010); Oligonucleotide Synthesis (M.J. Gait, ed., 1984); Methods in Molecular Biology, Humana Press; Cell Biology: A Laboratory Notebook (J.E. Cellis, ed., Academic Press, 1998); Introduction to Cell and Tissue Culture (J.P. Mather and P.E. Roberts, Plenum Press, 1998); Cell and Tissue Culture: Laboratory Procedures (A. Doyle, J.B. Griffiths, and D.G. Newell, eds., J. Wiley and Sons, 1993-8); Handbook of Experimental Immunology (D.M. Weir and C.C. Blackwell, eds., 1996); Gene Transfer Vectors for Mammalian Cells (J.M. Miller and M.P. Calos, eds., 1987); PCR: The Polymerase Chain Reaction, (Mullis et al., eds., 1994); Current Protocols in Immunology (J.E. Coligan et al., eds., 1991); Short Protocols in Molecular Biology (Ausubel et al., eds., J. Wiley and Sons, 2002); Immunobiology (C.A. Janeway et al., 2004); Antibodies (P. Finch, 1997); Antibodies: A Practical Approach (D. Catty., ed., IRL Press, 1988-1989); Monoclonal Antibodies: A Practical Approach (P. Shepherd and C. Dean, eds., Oxford University Press, 2000); Using Antibodies: A Laboratory Manual (E. Harlow and D. Lane, Cold Spring Harbor Laboratory Press, 1999); The Antibodies (M. Zanetti and J. D. Capra, eds., Harwood Academic Publishers, 1995); and Cancer: Principles and Practice of Oncology (V.T. DeVita et al., eds., J.B. Lippincott Company, 2011).

### II. Definitions

A "vector," as used herein, refers to a recombinant plasmid or virus that comprises a nucleic acid to be delivered into a host cell, either in vitro or in vivo.

The term "polynucleotide" or "nucleic acid" as used herein refers to a polymeric form of nucleotides of any length, either ribonucleotides or deoxyribonucleotides. Thus, this term includes, but is not limited to, single-, double- or multi-stranded DNA or RNA, genomic DNA, cDNA, DNA-RNA hybrids, or a polymer comprising purine and pyrimidine bases, or other natural, chemically or biochemically modified, non-natural, or derivatized nucleotide bases. The backbone of the nucleic acid can comprise sugars and phosphate groups (as may typically be found in RNA or DNA), or modified or substituted sugar or phosphate groups. Alternatively, the backbone of the nucleic acid can comprise a polymer of synthetic subunits such as phosphoramidates and thus can be an oligodeoxynucleoside phosphoramidate (P-NH₂) or a mixed phosphoramidate- phosphodiester oligomer. In addition, a double-stranded nucleic acid can be obtained from the single stranded polynucleotide product of chemical synthesis either by synthesizing the complementary strand and annealing the strands under appropriate conditions, or by synthesizing the complementary strand de novo using a DNA polymerase with an appropriate primer.

The terms "polypeptide" and "protein" are used interchangeably to refer to a polymer of amino acid residues, and are not limited to a minimum length. Such polymers of amino acid residues may contain natural or non-natural amino acid residues, and include, but are not limited to, peptides, oligopeptides, dimers, trimers, and multimers of amino acid residues. Both full-length proteins and fragments thereof are encompassed by the definition. The terms also include post-expression modifications of the polypeptide, for example, glycosylation, sialylation, acetylation, phosphorylation, and the like. Furthermore, for purposes of the present invention, a "polypeptide" refers to a protein which includes modifications, such as deletions, additions, and substitutions (generally conservative in nature), to the native sequence, as long as the protein maintains the desired activity. These modifications may be deliberate, as through site-directed mutagenesis, or may be accidental, such as through mutations of hosts which produce the proteins or errors due to PCR amplification.

A "recombinant viral vector" refers to a recombinant polynucleotide vector comprising one or more heterologous sequences (*i.e.*, nucleic acid sequence not of viral origin). In the case of recombinant AAV vectors, the recombinant nucleic acid is flanked by at least one, *e.g.,* two, inverted terminal repeat sequences (ITRs).

A "recombinant AAV vector (rAAV vector)" refers to a polynucleotide vector comprising one or more heterologous sequences (*i.e.*, nucleic acid sequence not of AAV origin) that are flanked by at least one, *e.g.,* two, AAV inverted terminal repeat sequences (ITRs). Such rAAV vectors can be replicated and packaged into infectious viral particles when present in a host cell that has been infected with a suitable helper virus (or that is expressing suitable helper functions) and that is expressing AAV rep and cap gene products (i.e. AAV Rep and Cap proteins). When a rAAV vector is incorporated into a larger polynucleotide (e.g., in a chromosome or in another vector such as a plasmid used for cloning or transfection), then the rAAV vector may be referred to as a "pro-vector" which can be "rescued" by replication and encapsidation in the presence of AAV packaging functions and suitable helper functions. A rAAV vector can be in any of a number of forms, including, but not limited to, plasmids, linear artificial chromosomes, complexed with lipids, encapsulated within liposomes, and, in embodiments, encapsidated in a viral particle, particularly an AAV particle. A rAAV vector can be packaged into an AAV virus capsid to generate a "recombinant adeno-associated viral particle (rAAV particle)".

An "rAAV virus" or "rAAV viral particle" refers to a viral particle composed of at least one AAV capsid protein and an encapsidated rAAV vector genome.

"Heterologous" means derived from a genotypically distinct entity from that of the rest of the entity to which it is compared or into which it is introduced or incorporated. For example, a nucleic acid introduced by genetic engineering techniques into a different cell type is a heterologous nucleic acid (and, when expressed, can encode a heterologous polypeptide). Similarly, a cellular sequence (e.g., a gene or portion thereof) that is incorporated into a viral vector is a heterologous nucleotide sequence with respect to the vector.

The term "transgene" refers to a nucleic acid that is introduced into a cell and is capable of being transcribed into RNA and optionally, translated and/or expressed under appropriate conditions. In aspects, it confers a desired property to a cell into which it was introduced, or otherwise leads to a desired therapeutic or diagnostic outcome. In another aspect, it may be transcribed into a molecule that mediates RNA interference, such as siRNA.

The terms "genome particles (gp)," "genome equivalents," or "genome copies" as used in reference to a viral titer, refer to the number of virions containing the recombinant AAV DNA genome, regardless of infectivity or functionality. The number of genome particles in a particular vector preparation can be measured by procedures such as described in the Examples herein, or for example, in Clark et al. (1999) Hum. Gene Ther., 10:1031-1039; Veldwijk et al. (2002) Mol. Ther., 6:272-278.

The terms "infection unit (iu)," "infectious particle," or "replication unit," as used in reference to a viral titer, refer to the number of infectious and replication-competent recombinant AAV vector particles as measured by the infectious center assay, also known as replication center assay, as described, for example, in McLaughlin et al. (1988) J. Virol., 62:1963-1973.

The term "transducing unit (tu)" as used in reference to a viral titer, refers to the number of infectious recombinant AAV vector particles that result in the production of a functional transgene product as measured in functional assays such as described in Examples herein, or for example, in Xiao et al. (1997) Exp. Neurobiol., 144:113-124; or in Fisher et al. (1996) J. Virol., 70:520-532 (LFU assay).

An "inverted terminal repeat" or "ITR" sequence is a term well understood in the art and refers to relatively short sequences found at the termini of viral genomes which are in opposite orientation.

An "AAV inverted terminal repeat (ITR)" sequence, a term well-understood in the art, is an approximately 145-nucleotide sequence that is present at both termini of the native single-stranded AAV genome. The outermost 125 nucleotides of the ITR can be present in either of two alternative orientations, leading to heterogeneity between different AAV genomes and between the two ends of a single AAV genome. The outermost 125 nucleotides also contains several shorter regions of self-complementarity (designated A, A', B, B', C, C' and D regions), allowing intrastrand base-pairing to occur within this portion of the ITR.

A "terminal resolution sequence" or "trs" is a sequence in the D region of the AAV ITR that is cleaved by AAV rep proteins during viral DNA replication. A mutant terminal resolution sequence is refractory to cleavage by AAV rep proteins. "AAV helper functions" refer to functions that allow AAV to be replicated and packaged by a host cell. AAV helper functions can be provided in any of a number of forms, including, but not limited to, helper virus or helper virus genes which aid in AAV replication and packaging. Other AAV helper functions are known in the art such as genotoxic agents.

"AAV helper functions" refer to functions that allow AAV to be replicated and packaged by a host cell. AAV helper functions can be provided in any of a number of forms, including, but not limited to, helper virus or helper virus genes which aid in AAV replication and packaging. Other AAV helper functions are known in the art such as genotoxic agents.

A "helper virus" for AAV refers to a virus that allows AAV (which is a defective parvovirus) to be replicated and packaged by a host cell. A number of such helper viruses have been identified, including adenoviruses, herpesviruses, poxviruses such as vaccinia, and baculovirus. The adenoviruses encompass a number of different subgroups, although Adenovirus type 5 of subgroup C (Ad5) is most commonly used. Numerous adenoviruses of human, non-human mammalian and avian origin are known and are available from depositories such as the ATCC. Viruses of the herpes family, which are also available from depositories such as ATCC, include, for example, herpes simplex viruses (HSV), Epstein-Barr viruses (EBV), cytomegaloviruses (CMV) and pseudorabies viruses (PRV). Baculoviruses available from depositories include *Autographa californica* nuclear polyhedrosis virus.

"Percent (%) sequence identity" with respect to a reference polypeptide or nucleic acid sequence is defined as the percentage of amino acid residues or nucleotides in a candidate sequence that are identical with the amino acid residues or nucleotides in the reference polypeptide or nucleic acid sequence, after aligning the sequences and introducing gaps, if necessary, to achieve the maximum percent sequence identity, and not considering any conservative substitutions as part of the sequence identity. Alignment for purposes of determining percent amino acid or nucleic acid sequence identity can be achieved in various ways that are within the skill in the art, for instance, using publicly available computer software programs, for example, those described in Current Protocols in Molecular Biology (Ausubel et al., eds., 1987), Supp. 30, section 7.7.18, Table 7.7.1, and including BLAST, BLAST-2, ALIGN or Megalign (DNASTAR) software. A potential alignment program is ALIGN Plus (Scientific and Educational Software, Pennsylvania). Those skilled in the art can determine appropriate parameters for measuring alignment, including any algorithms needed to achieve maximal alignment over the full length of the sequences being compared. For purposes herein, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows: 100 times the fraction X/Y, where X is the number of amino acid residues scored as identical matches by the sequence alignment program in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. For purposes herein, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows: 100 times the fraction W/Z, where W is the number of nucleotides scored as identical matches by the sequence alignment program in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C.

An "isolated" molecule (*e.g.*, nucleic acid or protein) or cell means it has been identified and separated and/or recovered from a component of its natural environment.

An "effective amount" is an amount sufficient to effect beneficial or desired results, including clinical results (*e.g.*, amelioration of symptoms, achievement of clinical endpoints, and the like). An effective amount can be administered in one or more administrations. In terms of a disease state, an effective amount is an amount sufficient to ameliorate, stabilize, or delay development of a disease. For example, an effective amount of a rAAV particle expresses a desired amount of heterologous nucleic acid such as a therapeutic polypeptide or therapeutic nucleic acid.

An "individual" or "subject" is a mammal. Mammals include, but are not limited to, domesticated animals (*e.g.,* cows, sheep, cats, dogs, and horses), primates *(e.g.,* humans and non-human primates such as monkeys), rabbits, and rodents (*e.g.*, mice and rats). In certain embodiments, the individual or subject is a human.

As used herein, "treatment" is an approach for obtaining beneficial or desired clinical results. For purposes of this invention, beneficial or desired clinical results include, but are not limited to, alleviation of symptoms, diminishment of extent of disease, stabilized (*e.g.*, not worsening) state of disease, preventing spread (*e.g.*, metastasis) of disease, delay or slowing of disease progression, amelioration or palliation of the disease state, and remission (whether partial or total), whether detectable or undetectable. "Treatment" can also mean prolonging survival as compared to expected survival if not receiving treatment.

When used in reference to a gene or coding sequence, "N-acetylglucosamine-1-phosphotransferase (aka GlcNAc-1-phosphotransferase or GNPTAB)" refers to a polynucleotide sequence encoding the alpha and beta subunits of the enzyme that catalyzes a chemical reaction involving the formation of lysosomal-enzyme N-acetyl-glucosaminyl-phospho-D-mannose and UMP from UDP-N-acetyl-D-glucosamine and lysosomal-enzyme D-mannose (EC code 2.7.8.17). When used in reference to a polypeptide, "N-acetylglucosamine-1-phosphotransferase (aka GlcNAc-1-phosphotransferase or GNPTAB)" refers to the alpha and beta subunits of aforementioned enzyme (Kudo, M. et al., J Biol Chem. 2005, 280(43):36141-9; Gelfman, CM et al., Invest. Opthamol. Vis. Sci. 2007, 48(11):5221-5228). The full GlcNAc-1-phosphotransferase enzyme complex is known to include α₂, β₂, and γ₂ subunits, of which the alpha and beta subunits are required for catalytic activity. Any enzyme known or predicted to catalyze the reaction described by EC code 2.7.8.17 and/or perform the molecular function described by GO term GO: 0003976 may be a GNPTAB of the present disclosure. In some embodiments, the GNPTAB is a variant GNPTAB. In some embodiments, the GNPTAB is a truncated GNPTAB. In some embodiments, nucleic acid encoding the GNPTAB is about 4.7 kb. In some embodiments, nucleic acid encoding the GNPTAB is less than about 4.7 kb. In some embodiments, the variant (*e.g.*, truncated) GNPTAB comprises the alpha and beta subunits. In some embodiments, the variant GNPTAB (*e.g.,* a truncated GNPTAB) is at least about 70%, 75%, 80%, 85%, 90%, 91%, 92%, 93%, 94%, 95%, 96%, 97%, 98%, or 99%, identical to native GNPTAB. In some embodiments, the variant GNPTAB *(e.g.,* a truncated GNPTAB) maintains at least about 90*%*, 80%, 70%, 60*%*, 50%, 40*%*, 30*%*, 20%, or 10% the activity of native GNPTAB. Examples of human GNPTAB are provided by GenBank Accession Nos. NP_077288.2 and NM_024312.4. An example of a human GNPTAB amino acid sequence is provided by SEQ ID NO:1. Examples of mouse GNPTAB are provided by GenBank Accession No. NP_001004164. An example of a mouse GNPTAB amino acid sequence is provided by SEQ ID NO:2. Additional examples of GNPTAB are provided by GenBank Accession Nos. XP_001155334 and XP_509312 (*Pan troglodytes*), XP_002687680 and NP_001179157 (*Bos taurus*), XP_416329 (*Gallus gallus*), XP_532667 (*Canis familiaris*), XP_001497199 (*Equus caballus*), XP_001079967 and XP_343195 (*Rattus norviegicus*) and NP_001038233 (*Danio rerio*).

"Mucolipidosis type II" (the terms MLII, ML-II, and ML type II may be used interchangeably herein) and "mucolipidosis type III" (the terms MLIII, ML-III, and ML type III may be used interchangeably herein) refer to a class of diseases caused by mutations in the GNPTAB gene. Both diseases are autosomal recessive disorders. However, MLIII is typically associated with mutations causing a more mild loss of GNPTAB function as compared to MLII. As such, MLII typically results in more severe disease phenotypes than MLIII. MLII is also known as I-cell disease. Further description of MLII may be found in OMIM entry #252500. MLIII is also known as pseudo-Hurler polydystrophy. Further description of MLIII may be found in OMIM entry #252600.

"Chicken β-actin (CBA) promoter" refers to a polynucleotide sequence derived from a chicken β-actin gene (*e.g., Gallus gallus* beta actin, represented by GenBank Entrez Gene ID 396526). As used herein, "chicken β-actin promoter" may refer to a promoter containing a cytomegalovirus (CMV) early enhancer element, the promoter and first exon and intron of the chicken β-actin gene, and the splice acceptor of the rabbit beta-globin gene, such as the sequences described in Miyazaki, J., et al. (1989) Gene 79(2):269-77. As used herein, the term "CAG promoter" may be used interchangeably. As used herein, the term "CMV early enhancer/chicken beta actin (CAG) promoter" may be used interchangeably.

A shortened chicken beta actin promoter was chosen based on deletion studies that showed that sequences upstream of -106 could be deleted without significantly affecting promoter activity (Quitschke et al., J. Biol. Chem. 264:9539-9546, 1989).

Reference to "about" a value or parameter herein includes (and describes) embodiments that are directed to that value or parameter *per se.* For example, description referring to "about X" includes description of "X."

As used herein, the singular form of the articles "a," "an," and "the" includes plural references unless indicated otherwise.

It is understood that aspects and embodiments of the invention described herein include "comprising," "consisting," and/or "consisting essentially of" aspects and embodiments.

### III. Vectors

In certain aspects, the disclosure provides rAAV vectors, *e.g.*, suitable for use in any of the methods, rAAV particles, and/or pharmaceutical compositions described herein. For example, in some aspects, a heterologous nucleic acid *(e.g.,* a polynucleotide sequence encoding a functional GNPTAB polypeptide) is delivered to a subject through a rAAV vector of the present disclosure.

Certain aspects of the present disclosure relate to N-acetylglucosamine-1-phosphate transferase, alpha and beta subunits (GNPTAB), *e.g.*, GNPTAB polypeptides, or nucleic acids encoding a GNPTAB polypeptide. As is known in the art, the N-acetylglucosamine-1-phosphate transferase (also known as N-acetylglucosamine-1-phosphotransferase) enzyme includes two alpha, two beta, and two gamma subunits. The alpha and beta subunits are encoded by a GNPTAB gene (also known as *GNPTA*, I-cell disease or *ICD*, or *EG432486* or *mKIAA1208* in mouse). Examples of GNPTAB genes include, for example, human GNPTAB *(e.g.,* as described by NCBI Gene ID No. 79158) and mouse GNPTAB *(e.g.,* as described by NCBI Gene ID No. 432486).

In some embodiments, the GNPTAB polypeptide may be a human GNPTAB polypeptide. A human GNPTAB polypeptide sequence may include without limitation NCBI Reference Sequence No. NP_077288. In some embodiments, the GNPTAB polypeptide comprises the amino acid sequence of SEQ ID NO: 1. In some embodiments, the GNPTAB polypeptide comprises an amino acid sequence that is at least about 80%, at least about 81%, at least about 82%, at least about 83%, at least about 84%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, or at least about 99% identical to the amino acid sequence of SEQ ID NO: 1. In some embodiments, the GNPTAB is a truncated GNPTAB. In some embodiments, nucleic acid encoding the GNPTAB is about 4.7 kb. In some embodiments, nucleic acid encoding the GNPTAB is less than about 4.7 kb. In some embodiments, the variant (*e.g.*, truncated) GNPTAB comprises the alpha and beta subunits. In some embodiments, the GNPTAB polypeptide is a variant GNPTAB polypeptide (*e.g*., truncated GNPTAB) that maintains at least a portion of the activity of a wild-type GNPTAB polypeptide (*e.g.*, at least about any of 5%, 10%, 25%, 50%, 75% or 100% of wild-type GNPTAB activity). In some embodiments, the variant GNPTAB polypeptide (*e.g.*, truncated GNPTAB) has greater activity compared to a wild-type GNPTAB polypeptide (*e.g.*, at least about any of 125%, 150%, 200%, 300%, or 500% greater activity compared to wild-type GNPTAB).

According to the invention, the heterologous nucleic acid *(e.g.,* nucleic acid encoding GNPTAB) is operably linked to a promoter. According to the invention, the promoter comprises a truncated CBA promoter. According to the invention, the promoter comprises a shortened CMV enhancer.

According to the invention, the vector comprises an intron. The intron is a minute virus of mice (MVM) intron.

In some embodiments, the vector comprises a polyadenylation (polyA) sequence. Numerous examples of polyadenylation sequences are known in the art, such as a bovine growth hormone (BGH) Poly(A) sequence (see, *e.g.*, accession number EF592533), an SV40 polyadenylation sequence, and an HSV TK pA polyadenylation sequence.

Without wishing to be bound to theory, because of the large size of the GNPTAB coding sequence, it may be advantageous to minimize the size of other elements of the rAAV vector (*e.g.*, a promoter, enhancer, intron, polyA sequence, and so forth). In some embodiments, a shortened variant of the CBA promoter described herein may be used in a rAAV vector. Methods for generating a shortened variant of a promoter are known in the art. For example, the promoter of interest could be mutated by introducing deletions of one or more nucleotides in the promoter sequence, and such variant promoter sequences could be individually cloned into vectors that include a reporter construct under the regulation of each promoter sequence. This system could be used to identify shortened variants of a promoter that retain a designated strength (e.g., amount of transcript produced). A rAAV vector according to the present invention includes truncated, and/or shortened CMV enhancer/CBA promoter, *e.g.*, as described herein. Similar methods could be used to identify shortened variants of an intron that retain proper levels of transcription, mRNA stability, and/or splicing. In some embodiments, a rAAV vector of the present disclosure may include a shortened intron as described herein. Similar methods could be used to identify shortened variants of a polyA sequence that retain proper levels of transcription, mRNA stability, and/or polyadenylation. In some embodiments, a rAAV vector of the present disclosure may include a shortened polyA sequence as described herein. In some embodiments the GTNAP gene comprises a nucleotide sequence encoding the amino acid sequence of SEQ ID NO: 1 or 2.

The present application discloses the use of a recombinant viral genome for introduction of one or more nucleic acid sequences encoding a therapeutic polypeptide and/or nucleic acid for packaging into a rAAV viral particle. The recombinant viral genome may include any element to establish the expression of the therapeutic polypeptide and/or nucleic acid, for example, a promoter, an ITR of the present disclosure, a ribosome binding element, terminator, enhancer, selection marker, intron, polyA signal, and/or origin of replication.

### IV. Viral particles and methods of producing viral particles

Certain aspects of the present disclosure relate to rAAV particles, *e.g.*, containing a rAAV vector of the present disclosure. In an AAV particle, a nucleic acid is encapsidated in the AAV particle. The AAV particle also comprises capsid proteins. In some embodiments, the nucleic acid comprises the coding sequence(s) of interest (*e.g.*, a GNPTAB coding sequence) operatively linked components in the direction of transcription, control sequences including transcription initiation and termination sequences, thereby forming an expression cassette. The expression cassette is flanked on the 5' and 3' end by at least one functional AAV ITR sequence. By "functional AAV ITR sequences" it is meant that the ITR sequences function as intended for the rescue, replication and packaging of the AAV virion. *See* Davidson et al., PNAS, 2000, 97(7)3428-32; Passini et al., J. Virol., 2003, 77(12):7034-40; and Pechan et al., Gene Ther., 2009, 16:10-16. For practicing some aspects of the invention, the recombinant vectors comprise at least all of the sequences of AAV essential for encapsidation and the physical structures for infection by the rAAV. AAV ITRs for use in the vectors of the invention need not have a wild-type nucleotide sequence *(e.g.,* as described in Kotin, Hum. Gene Ther., 1994, 5:793-801), and may be altered by the insertion, deletion or substitution of nucleotides or the AAV ITRs may be derived from any of several AAV serotypes. More than 40 serotypes of AAV are currently known, and new serotypes and variants of existing serotypes continue to be identified. See Gao et al., PNAS, 2002, 99(18): 11854-6; Gao et al., PNAS, 2003, 100(10):6081-6; and Bossis et al., J. Virol., 2003, 77(12):6799-810. Use of any AAV serotype is considered within the scope of the present invention. In some embodiments, a rAAV vector is a vector derived from an AAV serotype, including without limitation, AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV DJ, a goat AAV, bovine AAV, or mouse AAV ITRs or the like. In some embodiments, the nucleic acid in the AAV comprises an ITR of AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV DJ, a goat AAV, bovine AAV, or mouse AAV ITRs or the like.

In some embodiments, a rAAV particle comprises an encapsidation protein selected from AAV1, AAV2, AAV3, AAV4, AAV5, AAV6 (*e.g.,* a wild-type AAV6 capsid, or a variant AAV6 capsid such as ShH10, as described in U.S. PG Pub. 2012/0164106), AAV7, AAV8, AAVrh8, AAVrh8R, AAV9 (*e.g.*, a wild-type AAV9 capsid, or a modified AAV9 capsid as described in U.S. PG Pub. 2013/0323226), AAV10, AAVrh10, AAV11, AAV12, a tyrosine capsid mutant, a heparin binding capsid mutant, an AAV2R471A capsid, an AAVAAV2/2-7m8 capsid, an AAV DJ capsid (*e.g.*, an AAV-DJ/8 capsid, an AAV-DJ/9 capsid, or any other of the capsids described in U.S. PG Pub. 2012/0066783), AAV2 N587A capsid, AAV2 E548A capsid, AAV2 N708A capsid, AAV V708K capsid, goat AAV capsid, AAV1/AAV2 chimeric capsid, bovine AAV capsid, mouse AAV capsid, rAAV2/HBoV1 capsid, or an AAV capsid described in U.S. Pat. No. 8,283,151 or International Publication No. WO/2003/042397. In further embodiments, a rAAV particle comprises capsid proteins of an AAV serotype from Clades A-F.

Different AAV serotypes are used to optimize transduction of particular target cells or to target specific cell types within a particular target tissue (*e.g.*, a diseased tissue). A rAAV particle can comprise viral proteins and viral nucleic acids of the same serotype or a mixed serotype. For example, a rAAV particle may contain one or more ITRs and capsid derived from the same AAV serotype, or a rAAV particle may contain one or more ITRs derived from a different AAV serotype than capsid of the rAAV particle. In certain embodiments, a rAAV particle contains an AAV8 capsid and one or more *(e.g.,* 2) AAV2 ITRs.

### Production of AAV particles

Numerous methods are known in the art for production of rAAV vectors, including transfection, stable cell line production, and infectious hybrid virus production systems which include adenovirus-AAV hybrids, herpesvirus-AAV hybrids (Conway, JE et al., (1997) J. Virology 71(11):8780-8789) and baculovirus-AAV hybrids (Urabe, M. et al., (2002) Human Gene Therapy 13(16):1935-1943; Kotin, R. (2011) Hum Mol Genet. 20(R1): R2-R6). rAAV production cultures for the production of rAAV virus particles all require; 1) suitable host cells, 2) suitable helper virus function, 3) AAV rep and cap genes and gene products; 4) a nucleic acid (such as a therapeutic nucleic acid) flanked by at least one AAV ITR sequences (e.g., an AAV genome encoding GNPTAB); and 5) suitable media and media components to support rAAV production. In some embodiments, the suitable host cell is a primate host cell. In some embodiments, the suitable host cell is a human-derived cell lines such as HeLa, A549, 293, or Perc.6 cells. In some embodiments, the suitable helper virus function is provided by wild-type or mutant adenovirus (such as temperature sensitive adenovirus), herpes virus (HSV), baculovirus, or a plasmid construct providing helper functions. In some embodiments, the AAV rep and cap gene products may be from any AAV serotype. In general, but not obligatory, the AAV rep gene product is of the same serotype as the ITRs of the rAAV vector genome as long as the rep gene products may function to replicated and package the rAAV genome. Suitable media known in the art may be used for the production of rAAV vectors. These media include, without limitation, media produced by Hyclone Laboratories and JRH including Modified Eagle Medium (MEM), Dulbecco's Modified Eagle Medium (DMEM), custom formulations such as those described in U.S. Patent No. 6,566,118, and Sf-900 II SFM media as described in U.S. Patent No. 6,723,551. In some embodiments, the AAV helper functions are provided by adenovirus or HSV. In some embodiments, the AAV helper functions are provided by baculovirus and the host cell is an insect cell (*e.g.*, *Spodoptera frugiperda* (Sf9) cells).

One method for producing rAAV particles is the triple transfection method. Briefly, a plasmid containing a rep gene and a capsid gene, along with a helper adenoviral plasmid, may be transfected (*e.g.*, using the calcium phosphate method) into a cell line (*e.g.*, HEK-293 cells), and virus may be collected and optionally purified. As such, in some embodiments, the rAAV particle was produced by triple transfection of a nucleic acid encoding the rAAV vector, a nucleic acid encoding AAV rep and cap, and a nucleic acid encoding AAV helper virus functions into a host cell, wherein the transfection of the nucleic acids to the host cells generates a host cell capable of producing rAAV particles.

In some embodiments, rAAV particles may be produced by a producer cell line method (see Martin et al., (2013) Human Gene Therapy Methods 24:253-269; U.S. PG Pub. No. US2004/0224411; and Liu, X.L. et al. (1999) Gene Ther. 6:293-299). Briefly, a cell line (*e.g.,* a HeLa, 293, A549, or Perc.6 cell line) may be stably transfected with a plasmid containing a rep gene, a capsid gene, and a vector genome comprising a promoter-heterologous nucleic acid sequence (*e.g.*, GNPTAB). Cell lines may be screened to select a lead clone for rAAV production, which may then be expanded to a production bioreactor and infected with a helper virus (*e.g.*, an adenovirus or HSV) to initiate rAAV production. Virus may subsequently be harvested, adenovirus may be inactivated (*e.g.*, by heat) and/or removed, and the rAAV particles may be purified. As such, in some embodiments, the rAAV particle was produced by a producer cell line comprising one or more of nucleic acid encoding the rAAV vector, a nucleic acid encoding AAV rep and cap, and a nucleic acid encoding AAV helper virus functions. As described herein, the producer cell line method may be advantageous for the production of rAAV particles with an oversized genome, as compared to the triple transfection method.

In some embodiments, the nucleic acid encoding AAV rep and cap genes and/or the rAAV genome are stably maintained in the producer cell line. In some embodiments, nucleic acid encoding AAV rep and cap genes and/or the rAAV genome is introduced on one or more plasmids into a cell line to generate a producer cell line. In some embodiments, the AAV rep, AAV cap, and rAAV genome are introduced into a cell on the same plasmid. In other embodiments, the AAV rep, AAV cap, and rAAV genome are introduced into a cell on different plasmids. In some embodiments, a cell line stably transfected with a plasmid maintains the plasmid for multiple passages of the cell line (*e.g.*, 5, 10, 20, 30, 40, 50 or more than 50 passages of the cell). For example, the plasmid(s) may replicate as the cell replicates, or the plasmid(s) may integrate into the cell genome. A variety of sequences that enable a plasmid to replicate autonomously in a cell (*e.g.,* a human cell) have been identified (*see*, *e.g.*, Krysan, P.J. et al. (1989) Mol. Cell Biol. 9:1026-1033). In some embodiments, the plasmid(s) may contain a selectable marker (*e.g.*, an antibiotic resistance marker) that allows for selection of cells maintaining the plasmid. Selectable markers commonly used in mammalian cells include without limitation blasticidin, G418, hygromycin B, zeocin, puromycin, and derivatives thereof. Methods for introducing nucleic acids into a cell are known in the art and include without limitation viral transduction, cationic transfection (*e.g.*, using a cationic polymer such as DEAE-dextran or a cationic lipid such as lipofectamine), calcium phosphate transfection, microinjection, particle bombardment, electroporation, and nanoparticle transfection (for more details, *see e.g.*, Kim, T.K. and Eberwine, J.H. (2010) Anal. Bioanal. Chem. 397:3173-3178).

In some embodiments, the nucleic acid encoding AAV rep and cap genes and/or the rAAV genome are stably integrated into the genome of the producer cell line. In some embodiments, nucleic acid encoding AAV rep and cap genes and/or the rAAV genome is introduced on one or more plasmids into a cell line to generate a producer cell line. In some embodiments, the AAV rep, AAV cap, and rAAV genome are introduced into a cell on the same plasmid. In other embodiments, the AAV rep, AAV cap, and rAAV genome are introduced into a cell on different plasmids. In some embodiments, the plasmid(s) may contain a selectable marker (*e.g.*, an antibiotic resistance marker) that allows for selection of cells maintaining the plasmid. Methods for stable integration of nucleic acids into a variety of host cell lines are known in the art. For example, repeated selection (*e.g.*, through use of a selectable marker) may be used to select for cells that have integrated a nucleic acid containing a selectable marker (and AAV cap and rep genes and/or a rAAV genome). In other embodiments, nucleic acids may be integrated in a site-specific manner into a cell line to generate a producer cell line. Several site-specific recombination systems are known in the art, such as FLP/FRT (*see, e.g.*, O'Gorman, S. et al. (1991) Science 251:1351-1355), Cre/loxP (*see, e.g.,* Sauer, B. and Henderson, N. (1988) Proc. Natl. Acad. Sci. 85:5166-5170), and phi C31-att (*see, e.g.*, Groth, A.C. et al. (2000) Proc. Natl. Acad. Sci. 97:5995-6000).

In some embodiments, the producer cell line is derived from a primate cell line (*e.g.*, a non-human primate cell line, such as a Vero or FRhL-2 cell line). In some embodiments, the cell line is derived from a human cell line. In some embodiments, the producer cell line is derived from HeLa, 293, A549, or PERC.6^{®} (Crucell) cells. For example, prior to introduction and/or stable maintenance/integration of nucleic acid encoding AAV rep and cap genes and/or the oversized rAAV genome into a cell line to generate a producer cell line, the cell line is a HeLa, 293, A549, or PERC.6^{®} (Crucell) cell line, or a derivative thereof.

In some embodiments, the producer cell line is adapted for growth in suspension. As is known in the art, anchorage-dependent cells are typically not able to grow in suspension without a substrate, such as microcarrier beads. Adapting a cell line to grow in suspension may include, for example, growing the cell line in a spinner culture with a stirring paddle, using a culture medium that lacks calcium and magnesium ions to prevent clumping (and optionally an antifoaming agent), using a culture vessel coated with a siliconizing compound, and selecting cells in the culture (rather than in large clumps or on the sides of the vessel) at each passage. For further description, *see, e.g.*, ATCC frequently asked questions document (available at www.atcc.org/Global/FAQs/9/1/Adapting*%*20a*%*20monolayer*%*20cell*%*20line*%*20to*%*20susp ension-40.aspx) and references cited therein.

In some aspects, it is disclosed a method for producing any rAAV particle as disclosed herein comprising (a) culturing a host cell under a condition that rAAV particles are produced, wherein the host cell comprises (i) one or more AAV package genes, wherein each said AAV packaging gene encodes an AAV replication and/or encapsidation protein; (ii) a rAAV pro-vector comprising a nucleic acid encoding a heterologous nucleic acid as described herein flanked by at least one AAV ITR, and (iii) an AAV helper function; and (b) recovering the rAAV particles produced by the host cell. In some embodiments, said at least one AAV ITR is selected from the group consisting of AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV DJ, a goat AAV, bovine AAV, or mouse AAV serotype ITRs or the like. For example, in some embodiments, the AAV serotype is AAV1, AAV2, AAV5, AAV6, AAV7, AAV8, AAVrh8, AAVrh8R, AAV9, AAV10, or AAVrh10. In certain embodiments, the nucleic acid in the AAV comprises an AAV2 ITR. In some embodiments, said encapsidation protein is selected from the group consisting of AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV2/2-7m8, AAV DJ, AAV2 N587A, AAV2 E548A, AAV2 N708A, AAV V708K, goat AAV, AAV1/AAV2 chimeric, bovine AAV, or mouse AAV capsid rAAV2/HBoV1 serotype capsid proteins or mutants thereof. In some embodiments, the encapsidation protein is an AAV8 capsid protein. In some embodiments, the rAAV particles comprise an AAV8 capsid and a recombinant genome comprising AAV2 ITRs, and nucleic acid encoding a therapeutic transgene/nucleic acid (*e.g.,* nucleic acid encoding GNPTAB).

Suitable rAAV production culture media may be supplemented with serum or serum-derived recombinant proteins at a level of 0.5%-20% (v/v or w/v). Alternatively, as is known in the art, rAAV vectors may be produced in serum-free conditions which may also be referred to as media with no animal-derived products. One of ordinary skill in the art may appreciate that commercial or custom media designed to support production of rAAV vectors may also be supplemented with one or more cell culture components know in the art, including without limitation glucose, vitamins, amino acids, and or growth factors, in order to increase the titer of rAAV in production cultures.

rAAV production cultures can be grown under a variety of conditions (over a wide temperature range, for varying lengths of time, and the like) suitable to the particular host cell being utilized. As is known in the art, rAAV production cultures include attachment-dependent cultures which can be cultured in suitable attachment-dependent vessels such as, for example, roller bottles, hollow fiber filters, microcarriers, and packed-bed or fluidized-bed bioreactors. rAAV vector production cultures may also include suspension-adapted host cells such as HeLa, 293, and SF-9 cells which can be cultured in a variety of ways including, for example, spinner flasks, stirred tank bioreactors, and disposable systems such as the Wave bag system.

rAAV vector particles for use of the invention may be harvested from rAAV production cultures by lysis of the host cells of the production culture or by harvest of the spent media from the production culture, provided the cells are cultured under conditions known in the art to cause release of rAAV particles into the media from intact cells, as described more fully in U.S. Patent No. 6,566,118). Suitable methods of lysing cells are also known in the art and include for example multiple freeze/thaw cycles, sonication, microfluidization, and treatment with chemicals, such as detergents and/or proteases.

In a further embodiment, the rAAV particles are purified. The term "purified" as used herein includes a preparation of rAAV particles devoid of at least some of the other components that may also be present where the rAAV particles naturally occur or are initially prepared from. Thus, for example, isolated rAAV particles may be prepared using a purification technique to enrich it from a source mixture, such as a culture lysate or production culture supernatant. Enrichment can be measured in a variety of ways, such as, for example, by the proportion of DNase-resistant particles (DRPs) or genome copies (gc) present in a solution, or by infectivity, or it can be measured in relation to a second, potentially interfering substance present in the source mixture, such as contaminants, including production culture contaminants or in-process contaminants, including helper virus, media components, and the like.

In some embodiments, the rAAV production culture harvest is clarified to remove host cell debris. In some embodiments, the production culture harvest is clarified by filtration through a series of depth filters including, for example, a grade DOHC Millipore Millistak+ HC Pod Filter, a grade A1HC Millipore Millistak+ HC Pod Filter, and a 0.2 µm Filter Opticap XL1O Millipore Express SHC Hydrophilic Membrane filter. Clarification can also be achieved by a variety of other standard techniques known in the art, such as, centrifugation or filtration through any cellulose acetate filter of 0.2 µm or greater pore size known in the art.

In some embodiments, the rAAV production culture harvest is further treated with Benzonase^{®} to digest any high molecular weight DNA present in the production culture. In some embodiments, the Benzonase^{®} digestion is performed under standard conditions known in the art including, for example, a final concentration of 1-2.5 units/ml of Benzonase^{®} at a temperature ranging from ambient to 37°C for a period of 30 minutes to several hours.

rAAV particles may be isolated or purified using one or more of the following purification steps: equilibrium centrifugation; flow-through anionic exchange filtration; tangential flow filtration (TFF) for concentrating the rAAV particles; rAAV capture by apatite chromatography; heat inactivation of helper virus; rAAV capture by hydrophobic interaction chromatography; buffer exchange by size exclusion chromatography (SEC); nanofiltration; and rAAV capture by anionic exchange chromatography, cationic exchange chromatography, or affinity chromatography. These steps may be used alone, in various combinations, or in different orders. In some embodiments, the method comprises all the steps in the order as described below. Methods to purify rAAV particles are found, for example, in Xiao et al., (1998) Journal of Virology 72:2224-2232; US Patent Numbers 6,989,264 and 8,137,948; and WO 2010/148143.

### V. Methods of Treatment

The references to methods of treatment in the following paragraphs of this description are to be interpreted as references to the rAAV particles and pharmaceutical compositions of the present invention for use in a method for treatment of the human (or animal) body by therapy.

Certain aspects of the present disclosure relate to methods of treating mucolipidosis type II and/or mucolipidosis type III or increasing body size, increasing bone mineral content, or increasing bone mineral density in a mammal with mucolipidosis type II or mucolipidosis type III. These methods are based in part on the discovery described herein that AAV-mediated expression of GNPTAB can ameliorate symptoms of MLII, such as bone growth impairments, in a mouse model of the disease. As described above, both diseases are caused by loss-of-function mutations in the GNPTAB gene, which encodes the catalytic alpha and beta subunits of GlcNAc-1-phosphotransferase.

MLII is known to be an autosomal recessive disorder caused by mutations in GNPTAB. GNPTAB activity is required to add mannose-6-phosphate to proteins, thereby marking them for trafficking to the lysosome. In the absence of GNPTAB activity, lysosomal proteins (*e.g.*, lysosomal hydrolases) are instead secreted extracellularly. As a result, substances that are normally broken down in lysosomes, such as glycosaminoglycans, lipids, and oligosaccharides, accumulate in cells, leading to the presence of large inclusions. MLII has also been called I-cell disease, due to the presence of these inclusion-cells ("I-cells"), which are identifiable by microscope. The symptoms of MLII often present shortly after birth and may include skeletal abnormalities, short stature, weak muscle tone, lack of muscle tone (hypotonia), hernias (e.g., protruded abdomen, umbilical hernias), hip dislocation and joint deformities, cardiomegaly, thickening and insufficiency of the mitral valve, progressive mucosal thickening of the airways, coarse and/or noisy breathing, frequent respiratory infections, constipation, diarrhea, delays in the development of gross and fine motor skills, hearing loss, and developmental delays, particularly in speech and motor skills, cognitive deficits. These symptoms are debilitating for MLII patients, who typically are not able to walk independently and do not survive past childhood.

Like MLII, MLIII is known in the art as an autosomal recessive disorder caused by mutations in GNPTAB. However, compared with MLII, MLIII typically results from weaker loss-of-function mutations in GNPTAB and is thereby characterized by milder disease phenotypes. MLIII symptoms may not be fully apparent until 3-5 years of age and are quite variable in severity, with some patients living past 60 while others do not survive past childhood. MLIII symptoms typically include skeletal abnormalities, short stature, aortic valve disease, corneal clouding, mild organ enlargement, loss of mobility, and joint abnormalities. MLIII has also been called pseudo-Hurler polydistrophy. For more detailed description and specific mutations found in MLII and MLIII patients, see, *e.g.,* Paik, K.H., et al. (2005) Hum. Mutat. 26(4):308-14.

Various diagnostic tests for MLII and MLIII are known in the art. In some embodiments, MLII and/or MLIII may be diagnosed by measuring the activity of one or more lysosomal enzymes in a serum sample or other patient sample (*e.g.*, a skin sample or cultured fibroblasts). The activity of certain lysosomal enzymes in serum may be 5- to 20-fold higher in MLII patients than in normal patients. Similarly, in MLIII the serum activity of these enzymes may be elevated up to 10-fold. These enzymes may include without limitation beta-D-hexosaminidase (EC code 3.2.1.52), beta-D-glucuronidase (EC code 3.2.1.31), beta-D-galactosidase (EC code 3.2.1.23), alpha-L-fucosidase (EC code 3.2.1.51) and alpha-D-mannosidase (EC code 3.2.1.24). In contrast, these activities in cultured fibroblasts may be deficient. In some embodiments, N-acetylglucosamine-1-phosphotransferase activity may be measured in order to diagnose MLII or MLIII. In some embodiments, a patient whose sample shows an N-acetylglucosamine-1-phosphotransferase activity of less than 1% compared to activity from a normal patient sample may be diagnosed with MLII. In some embodiments, a patient whose sample shows an N-acetylglucosamine-1-phosphotransferase activity of between 1%-10% compared to activity from a normal patient sample may be diagnosed with MLII.

In some embodiments, MLII and/or MLIII may be diagnosed by sequencing the GNPTAB locus (*e.g.*, the coding sequence, promoter/intron sequences, or any other control sequences) for mutations. MLII and MLIII may also be characterized by increased urinary polysaccharides and/or urinary glycosaminoglycans, but these symptoms may be not specific to MLII and MLIII. In some embodiments, MLII and/or MLIII may be diagnosed prenatally by examining a chorionic sample, *e.g.,* a trophoblast biopsy (see, *e.g.,* Poenaru, L., et al. (1984) Am. J. Hum. Genet. 36(6):1379-85). As described above, in MLII, I-cells from a patient sample (*e.g.*, fibroblasts) may also be identified by microscopy.

In some embodiments, a treatment for MLII and/or MLIII (*e.g.*, a gene therapy vector of the present disclosure) may be tested for therapeutic efficacy. For example, in some embodiments, a treatment for MLII and/or MLIII may result in an increase in body height gain, bone mineral content, bone mineral density, or may result in an amelioration of one or more symptoms of MLII and/or MLIII as described herein. The effectiveness of rAAV administration can be monitored by several criteria as described herein. For example, after treatment in a subject using methods of the present invention, the subject may be assessed for *e.g.,* an improvement and/or stabilization and/or delay in the progression of one or more signs or symptoms of the disease state by one or more clinical parameters including those described herein. Examples of such tests are known in the art, and include objective as well as subjective (*e.g.*, subject reported) measures.

In some embodiments, expression of GNPTAB may be measured to monitor therapeutic efficacy. Measuring GNPTAB expression may refer to measuring GNPTAB mRNA and/or protein expression. Various methods for measuring mRNA and/or protein expression are known in the art, including without limitation qPCR, Northern blotting, RNA-seq, semi-quantitative PCR, Western blotting, mass spectrometry, ELISA, and so forth.

In some embodiments, the activity of one or more lysosomal enzymes, including without limitation beta-D-hexosaminidase (EC code 3.2.1.52), beta-D-glucuronidase (EC code 3.2.1.31), beta-D-galactosidase (EC code 3.2.1.23), alpha-L-fucosidase (EC code 3.2.1.51) and alpha-D-mannosidase (EC code 3.2.1.24), may be measured in a patient sample (*e.g.*, a serum sample) to monitor therapeutic efficacy. A decrease in lysosomal enzyme activity in serum may indicate efficacy.

In some embodiments, an improvement in joint or limb function may indicate efficacy. In some embodiments, an improvement in speech may indicate efficacy. In some embodiments, an improvement in motor function may indicate efficacy. In some embodiments, increased growth rate (e.g., an increase in body height gain) may indicate efficacy. In some embodiments, as described in the Examples herein, bone mineral density, bone mineral content, and/or growth (e.g., height) may be assessed for monitoring therapeutic efficacy. Methods for monitoring height are well known to one of skill in the art. Tests for monitoring bone mineral density and bone mineral content (e.g., bone densitometry tests) are also well known to one of skill in the art (e.g., as described herein) and may include without limitation dual-energy x-ray absorptiometry (DEXA) scan, peripheral dual-energy x-ray absorptiometry (P-DEXA) scan, dual photon absorptiometry (DPA), and a computed tomography (CT) scan.

In some embodiments, a treatment for MLII and/or MLIII *(e.g.,* a gene therapy vector of the present disclosure) may be tested in an animal model. Animal models for MLII and MLIII are known in the art. In some embodiments, a treatment for MLII and/or MLIII may be tested in a mouse model, such as the genetically modified mouse model described in the Examples herein. In some embodiments, a treatment for MLII may be tested in a feline model for MLII (see Mazrier, H., et al. (2003) J. Hered. 94(5):363-73 or Bosshard, N.U., et al. (1996) Vet. Pathol. 33(1): 1-13 for further descriptions).

The selection of a particular rAAV vector and composition depend on a number of different factors, including, but not limited to, the individual human's medical history and features of the condition and the individual being treated. The assessment of such features and the design of an appropriate therapeutic regimen is ultimately the responsibility of the prescribing physician.

In some aspects, the invention provides a rAAV particle of the present disclosure for use in methods of treating MLII and/or MLIII. rAAV may be administered to a particular tissue of interest, or it may be administered systemically. In some embodiments, an effective amount of rAAV may be administered parenterally. Parenteral routes of administration may include without limitation intravenous, intraperitoneal, intraosseous, intra-arterial, intracerebral, intramuscular, intrathecal, subcutaneous, intracerebroventricular, intrahepatic, and so forth. In some embodiments, an effective amount of rAAV may be administered through one route of administration. In some embodiments, an effective amount of rAAV may be administered through a combination of more than one route of administration. In some embodiments, an effective amount of rAAV is administered to one location. In other embodiments, an effective amount of rAAV may be administered to more than one location.

An effective amount of rAAV (in some embodiments in the form of particles) is administered, depending on the objectives of treatment. For example, where a low percentage of transduction can achieve the desired therapeutic effect, then the objective of treatment is generally to meet or exceed this level of transduction. In some instances, this level of transduction can be achieved by transduction of only about 1 to 5% of the target cells of the desired tissue type, in some embodiments at least about 20% of the cells of the desired tissue type, in some embodiments at least about 50%, in some embodiments at least about 80%, in some embodiments at least about 95%, in some embodiments at least about 99% of the cells of the desired tissue type. The rAAV composition may be administered by one or more administrations, either during the same procedure or spaced apart by days, weeks, months, or years. One or more of any of the routes of administration described herein may be used. In some embodiments, multiple vectors may be used to treat the human.

Methods to identify cells transduced by AAV viral particles are known in the art; for example, immunohistochemistry or the use of a marker such as enhanced green fluorescent protein can be used to detect transduction of viral particles; for example viral particles comprising a rAAV capsid with one or more substitutions of amino acids.

In some embodiments an effective amount of rAAV particles is administered to more than one location simultaneously or sequentially. In other embodiments, an effective amount of rAAV particles is administered to a single location more than once (*e.g.,* repeated). In some embodiments, multiple injections of rAAV viral particles are no more than one hour, two hours, three hours, four hours, five hours, six hours, nine hours, twelve hours or 24 hours apart.

An effective amount of rAAV (according to the invention, in the form of particles) is administered, depending on the objectives of treatment. For example, where a low percentage of transduction can achieve the desired therapeutic effect, then the objective of treatment is generally to meet or exceed this level of transduction. In some instances, this level of transduction can be achieved by transduction of only about 1 to 5% of the target cells, in some embodiments at least about 20% of the cells of the desired tissue type, in some embodiments at least about 50%, in some embodiments at least about 80%, in some embodiments at least about 95%, in some embodiments at least about 99% of the cells of the desired tissue type. The rAAV composition may be administered by one or more administrations, either during the same procedure or spaced apart by days, weeks, months, or years. In some embodiments, multiple vectors may be used to treat the mammal (*e.g.*, a human).

In some embodiments, a rAAV composition of the present disclosure may be used for administration to a human. In some embodiments, a rAAV composition of the present disclosure may be used for pediatric administration. Without wishing to be bound to theory, because many of the symptoms of MLII and MLIII are developmental in nature (*e.g.*, growth, limb, and joint defects; speech and motor delays), it may be particularly advantageous to treat MLII and/or MLIII as early in life as possible. In some embodiments, an effective amount of rAAV (according to the invention, in the form of particles) is administered to a patient that is less than one month, less than two months, less than three months, less than four months, less than five months, less than six months, less than seven months, less than eight months, less than nine months, less than ten months, less than eleven months, less than one year, less than 13 months, less than 14 months, less than 15 months, less than 16 months, less than 17 months, less than 18 months, less than 19 months, less than 20 months, less than 21 months, less than 22 months, less than two years, or less than three years old.

In some embodiments, a rAAV composition of the present disclosure may be used for administration to a young adult. In some embodiments, an effective amount of rAAV (according to the invention in the form of particles) is administered to a patient that is less than 12 years old, less than 13 years old, less than 14 years old, less than 15 years old, less than 16 years old, less than 17 years old, less than 18 years old, less than 19 years old, less than 20 years old, less than 21 years old, less than 22 years old, less than 23 years old, less than 24 years old, or less than 25 years old.

### VI. Kits or Articles of Manufacture

The rAAV vectors, particles, and/or pharmaceutical compositions as described herein may be contained within a kit or article of manufacture, *e.g.,* designed for use in one of the methods of the invention as described herein.

Generally, the system comprises a cannula, one or more syringes (*e.g.*, 1, 2, 3, 4 or more), and one or more fluids (*e.g.*, 1, 2, 3, 4 or more) suitable for use in the methods of the invention.

The syringe may be any suitable syringe, provided it is capable of being connected to the cannula for delivery of a fluid. In some embodiments, the system has one syringe. In some embodiments, the system has two syringes. In some embodiments, the system has three syringes. In some embodiments, the system has four or more syringes. The fluids suitable for use in the methods of the invention include those described herein, for example, one or more fluids each comprising an effective amount of one or more vectors as described herein, and one or more fluids comprising one or more therapeutic agents.

In some embodiments, the kit comprises a single fluid (*e.g.,* a pharmaceutically acceptable fluid comprising an effective amount of the vector). In some embodiments, the kit comprises 2 fluids. In some embodiments, the kit comprises 3 fluids. In some embodiments, the kit comprises 4 or more fluids. A fluid may include a diluent, buffer, excipient, or any other liquid described herein or known in the art suitable for delivering, diluting, stabilizing, buffering, or otherwise transporting a rAAV vector composition of the present disclosure. In some embodiments, the kit comprises one or more buffers, *e.g.,* an aqueous pH buffered solution. Examples of buffers may include without limitation phosphate, citrate, Tris, HEPES, and other organic acid buffers.

In some embodiments, the kit comprises a container. Suitable containers may include, *e.g.*, vials, bags, syringes, and bottles. The container may be made of one or more of a material such as glass, metal, or plastic. In some embodiments, the container is used to hold a rAAV composition of the present disclosure. In some embodiments, the container may also hold a fluid and/or other therapeutic agent.

In some embodiments, the kit comprises an additional therapeutic agent with a rAAV composition of the present disclosure. In some embodiments, the rAAV composition and the additional therapeutic agent may be mixed. In some embodiments, the rAAV composition and the additional therapeutic agent may be kept separate. In some embodiments, the rAAV composition and the additional therapeutic agent may be in the same container. In some embodiments, the rAAV composition and the additional therapeutic agent may be in different containers. In some embodiments, the rAAV composition and the additional therapeutic agent may be administered simultaneously. In some embodiments, the rAAV composition and the additional therapeutic agent may be administered on the same day. In some embodiments, the rAAV composition may be administered within one day, two days, three days, four days, five days, six days, seven days, two weeks, three weeks, four weeks, two months, three months, four months, five months, or six months of administration of the additional therapeutic agent.

In some embodiments, the kit comprises a therapeutic agent to transiently suppress the immune system prior to AAV administration. In some embodiments, patients are transiently immune suppressed shortly before and after injection of the virus to inhibit the T cell response to the AAV particles (*e.g. see* Ferreira et al., Hum. Gene Ther. 25:180-188, 2014). In some embodiments, the kit further provides cyclosporine, mycophenolate mofetil, and/or methylprednisolone.

The rAAV particles and/or compositions of the invention may further be packaged into kits including instructions for use. In some embodiments, the kits further comprise a device for delivery (e.g., any type of parenteral administration described herein) of compositions of rAAV particles. In some embodiments, the instructions for use include instructions according to one of the methods described herein. In some embodiments, the instructions are printed on a label provided with (*e.g.*, affixed to) a container. In some embodiments, the instructions for use include instructions for administering to a mammal (e.g., a human) an effective amount of rAAV particles, *e.g.,* for treating mucolipidosis type II (ML II) and/or mucolipidosis type III (ML III), increasing body size, increasing bone mineral content, and/or increasing bone mineral density.

### VII. Non-Human Animal Models (not part of the invention)

The application discloses non-human animal models of Mucolipidosis II. GNPTAB mutant mice that may be generated by microinjection of embryonic stem (ES) cell clones into host blastocysts using standard methods. Briefly, targeted disruption of the mouse GNPTAB locus (e.g., from exon 12 to exon 20 was deleted) may be performed by homologous recombination with a replacement vector containing a reporter or selectable marker gene. All offsprings were genotyped by polymerase chain reaction analysis in tail snip DNA. All mice used in this study were males identified as wild type (+/+) mice or heterozygous (+/-) or homozygous (-/-). In some aspects, at least one allele of the GNPTAB gene comprises a deletion located between exons 12 and exon 20. In some aspects, at least one allele of the GNPTAB gene comprises a deletion spanning exons 12 and exon 20. In some embodiments, the deletion comprises a deletion of one or more of exons 12, 13, 14, 15, 16, 17, 18, 19 or 20. In some aspects, a portion of the GNPTAB gene is replaced by a gene encoding a reporter and/or selectable marker. In some aspects, the selectable marker confers resistance to neomycin. In some aspects, the animal is a mammal (*e.g.*, a rodent, a rabbit, a cat, a dog, a pig, a monkey). In some aspects, the mammal is a rodent *(e.g.,* a mouse, a rat, a hamster, a guinea pig). In some aspects, the animal is immunocompetent or immunodeficient. In some aspects, the animal model comprises a genetically modified animal. Other mouse models of ML II are provided by Gelfman et al., (Invest. Optham. Visual Sci. 2007, 48:5221-5228) and Paton, L. et al., (J Biol Chem. 2014, 289(39):26709-21).

In some aspects, the application discloses a method for evaluating an agent for treatment of Mucolipidosis II (ML II) comprising administering the agent to the animal model as described herein, wherein amelioration one or more symptoms of ML II indicates the agent may provide beneficial treatment of ML II. In some aspects, the symptom of ML II is decreased body weight, decreased bone density, decreased bone mineral content, skeletal defects, cognitive deficits, delays in the development of gross and fine motor skills, hearing loss, lack of muscle tone, protruded abdomen, umbilical hernias, progressive mucosal thickening of the airways, frequent respiratory infections, thickening and insufficiency of the mitral valve, constipation and/or diarrhea. In some aspects, the agent is a small molecule, a polypeptide, an antibody, a nucleic acid or a recombinant viral particle.

### EXAMPLES

The invention will be more fully understood by reference to the following examples. They should not, however, be construed as limiting the scope of the invention. It is understood that the examples and embodiments described herein are for illustrative purposes only.

### Example 1: Generation and characterization of GNPTAB knockout mice

Mucolipidosis type II (ML type II or ML-II, aka I-cell disease; OMIM entry #252500) is an autosomal recessive lysosomal storage disorder caused by N-acetylglucosamine-1-phosphotransferase (GNPTAB) deficiency. The presence of numerous inclusion bodies in the cytoplasm of fibroblasts, a lack of mucopolysacchariduria, increased lysosomal enzyme activities in serum, and decreased GlcNAc-phosphotransferase activity are hallmarks of this disease. In order to study the pathology of ML type II, a GNPTAB knockout (KO) mouse was developed and characterized.

### Methods

### Generation of AAV2/8-GNPTAB constructs

The coding sequence of mouse GNPTAB was amplified and codon-optimized for expression in mice by Genescript (Piscataway, NJ, USA). Because of the large size of the GNPTAB cDNA and the limited capacity of AAV, the cDNA does not fit into any available vectors. Therefore a new expression cassette was designed that contains shortened versions of the CMV enhancer and chicken beta-actin promoter, as well as a very small intron. Expression of full-length GNPTAB mRNAwas confirmed by transient infection of HEK293 cells in vitro followed by quantitative RT-PCR. This AAV2/8-GNPTAB was purified, stored at -70°C, and used at a concentration of 2.5×10¹² DNase resistant particles/mL. The shortened enhancer and promoter sequences were used for these studies.

### Animals

GNPTAB mutant mice were generated by microinjection of embryonic stem (ES) cell clones into host blastocysts using standard methods. Briefly, targeted disruption of the mouse GNPTAB locus (from exon 12 to exon 20 was deleted) was performed by homologous recombination with a replacement vector containing the neomycin-resistance gene. Mice used in this study were of mixed genetic background (129/Sv and C57BL/6). All offsprings were genotyped by polymerase chain reaction analysis in tail snip DNA. All mice used in this study were males identified as wild type (+/+) mice or heterozygous (+/-) or homozygous (-/-).

Mice were housed in groups of 2-5 per cage in a colony room under a 12-hour light-dark cycle. Rodent diet (Harlan Teklad #8604, Madison, WI, USA) and water were available *ad libitum.* Care of animals was conducted in accordance with the guidelines described in the Guide for the Care and Use of Laboratory Animals (National Academy Press, Washington DC, 1996).

### Vector injections

A single bilateral i.v. injection of PBS or AAV-GNPTAB was made into 6-week-old GNPTAB KO mice. A group of wild type or heterozygous mice injected with PBS served as a control group. Each mouse injected with AAV-GNPTAB received 3×10¹¹ vector particles. To reduce or eliminate immune response, anti-CD40 ligand antibody (MR1) alone was injected according to protocol by Halbert el al., (1998) J. Virol. 72:9795-9805.

### Auxology

All mice were examined weekly by measuring total body weight (g) and body length (nose-to-anus distance, mm), using an electronic digital caliper.

### Histology

Wild type and GNPTAB^{-/-} mice at 10 mo. of age were sedated and perfused via the heart with PBS. Following the perfusion, the femurs were removed, fixed in 4% paraformaldehyde (PFA), decalcified with 0.5 M EDTA, and paraffin embedded. Femur sections (4 µM) were stained with hematoxlin-eosin. Aperio ScanScope AT (v101.0) was used for scanning of slide and analysis at 4°C. The tissues were embedded and cut for TEM or hematoxylin and eosin (H&E).

### Ultrastructural analysis

Wild type and KO mice were decapitated, and the salivary glands were removed and fixed by immersion in 2.5% glutaraldehyde in PBS overnight at 4°C. The salivary glands were dehydrated and then washed in PBS buffer for a further 30 min followed by post-fixation with 1*%* osmium tetroxide for 1 h at room temperature. The specimens were dehydrated through graded alcohol series and embedded in Epon 812. Semi-thin serial sections were cut at 2.5 µM (Leica ultracut UCT), stained with Toluidine Blue and observed under a light microscope. Transmission electron microscopy observation was performed with a Mirgagni microscope (FEI).

### Lysosomal enzyme assay

Blood was taken and samples were centrifuged 15 min (8000xg) and plasma was stored at -70°C. Plasma lysosomal enzyme activities were measured at the Department of Chemical Pathology, Samsung Medical Center, South Korea. In ML II, specific GlcNAc-phosphotransferase activity was not able to be measured. Thus, a diagnosis was made based on elevated plasma lysosomal enzyme.

### DEXA analysis

Bone mineral density (BMD), bone mineral content (BMC), and lean mass were measured in anesthetized mice with the pDEXA Sabre X-ray Bone Densitometer. After anesthesia was administered, the weight of each mouse was recorded, and the mouse was then placed in the DEXA scanner. For data analysis, a region comprising the entire body of the mouse was defined.

### Real-time PCR

Real-time polymerase chain reaction is performed to quantify the mRNA levels of GNPTAB using an ABI PRISM 7900HT system and TaqMan gene expression assays (Applied Biosystems, Foster City, CA). The mRNA levels are expressed relative to the level of GAPDH. The 2-^{ΔΔ}CT method is used to analyze the data using SDS2.3 software (Applied Biosystems).

### Statistical Analysis

GraphPad Prism Version 5 was used for statistical analysis and drawing figures and tables. Significance of differences was determined by an unpaired Student's t-test, and one-way ANOVA test. Unless otherwise specified, all data are presented as mean±SEM.

### Results

A gene trap strategy was used to generate GNPTAB KO mice. As illustrated in **FIG. 1A****,** homologous recombination with a replacement vector containing the neomycin-resistance gene between exon 12 and 20 was used to generate the mice. As shown in **FIG. 1B**, the presence of the gene trap in the GNPTAB gene of the ES clones was confirmed by Southern blot analysis.

A comprehensive phenotypic analysis was performed on wild type, heterozygous, and homozygous animals. KO mice could be visually distinguished from wild-type (WT) littermates by their small size. Mean body weight (**FIG. 2A**) and length (**FIG. 2B**) were significantly reduced in the KO mice. Consistent with their small size, the homozygous mice exhibited a coarse facial shape (**FIG. 2C**).

As shown in **FIG. 3A**, in normal cartilage, clear lacunar space surrounded chondrocytes. The cytoplasm contained a single, clear vacuole. In KO mice, femoral chondrocytes were markedly hypertrophic and completely filled their enlarged lacunae (**FIG**. **3B**). The cytoplasm of hypertrophic chondrocyte was distended by abundant microvacuoles containing scant amounts of finely granular amphophilic material. There was less fixation-related shrinkage of chondrocytes, which filled the enlarged lacunae. This contrasts with wild type chondrocytes, which contained a single large, clear vacuole and only partially filled lacunae.

The acini of the exocrine salivary glands, which consist of mucous- and serous-type secretory cells, exhibited extensive vacuolization in KO mice when examined by light microscopy (Gelfman et al., 2007, Invest. Optham. Visual Sci. 48:5221-5228). To gain insight into the underlying pathology, the submandibular salivary gland was analyzed by electron microscopy (EM).

Both mucous- and serous-type secretory cells were readily observed in the wild type mice (**FIG. 4A-C**). By contrast, the overall architecture of the submandibular salivary gland of the KO mice was so grossly disrupted that it greatly hampered the identification of the serous cells in the EM sections (**FIG. 4D-F**). The mucous-type secretory cells were packed with large membrane-bound vacuoles that contained heterogeneous material (**FIG. 4E****, F**). The large vacuoles were filled with un-degraded cytoplasmic material that accumulated in the KO mucous cells and were surrounded by a single membrane. These observations indicate that they likely represent autolysosomes formed by fusion of autophagic compartments with lysosomes.

Patients with ML-II have greatly elevated levels of lysosomal enzymes in their sera because of the inability to synthesize the mannose-6-phosphate recognition marker that is essential for proper targeting of these enzymes to lysosomes. This trafficking defect results in hypersecretion of the enzymes into the blood. **FIGS. 5A-5D** shows that compared with wild type mice, the KO mice exhibited great increased levels of lysosomal enzymes, such as N-acetylglucosaminidase (**FIG. 5A**), β-hexosaminidase A (**FIG. 5B**), β-galactosidase (**FIG. 5C**), and β-glucuronidase (**FIG. 5D**). This phenotype would be expected if GlcNAc-1-phosphotransferase activity were defective in the homozygous mice, consistent with observations in humans.

In summary, these experiments demonstrate that GNPTAB KO mice displayed clear phenotypic differences as compared to wild-type mice, particularly with respect to growth, salivary gland morphology, and lysosomal enzyme levels. These results indicate that GNPTAB KO mice represent a model system in which to study therapeutic treatments for ML-II.

### Example 2: Evaluation of AAV-mediated administration of GNPTAB in GNPTAB KO mice

Because patients with ML-II show growth retardation, a major goal of this study was to evaluate the efficacy for AAV-mediated GNPTAB administration that may promote growth in an ML-II model. Therefore, the phenotypes of wild-type, GNPTAB heterozygous, and GNPTAB KO mice were analyzed, as compared to GNPTAB KO mice injected with AAV-GNPTAB.

As shown in **FIGS. 6A** and **6B**, all analytical assessments were carried out during two periods post-injection: the first began 16 weeks post-injection (on 12 weeks old age), the second began 32 weeks post-injection (on 38 weeks old age). Auxological analysis was added 6 weeks post-injection.

A plasmid was constructed that contained an expression cassette expressing the mouse GNPTAB cDNA regulated by the CMV enhancer/CBA promoter and a BGH polyA signal (**FIG**. **7A**). Full length GNPTAB was under the control of the shortened CMV enhance/CBA promoter as described herein. Quantitative real-time PCR analyses of livers from GNPTAB KO mice injected with AAV2/8-GNPTAB indicated specific and highly expression of AAV-GNPTAB. As expected, any AAV-GNPTAB mRNA was absent in samples from control littermates (**FIG. 7B**).

As an initial test to determine whether AAV-mediated gene transfer affects metabolism in a physiologically relevant way, weight gain was monitored for 32 weeks post injection. As shown in **FIG. 8A**, no effect was observed for treatment of AAV-GNPTAB in KO mice. **FIG. 8B** shows that there was no difference observed between control and AAV-GNPTAB treated KO mice in terms of weight gained.

Next, the effectiveness of attenuating the dwarfism phenotype of KO mice was assessed. An improvement in dwarfism was noted after 6 weeks of therapy in KO mice injected AAV-GNPTAB, showing an increase in body size (**FIGS. 9A, 9B** and **Table 1** below).

**Table 1. Height change**

| Genotype | Starting height | Ratio of height increase (Height after indicated interval/starting height) | |
|---|---|---|---|
| | | Height after 6 weeks | Height after 32 weeks |
| | (mm) | | |
| Wild-type (+/+) | 84.31±0.885 | 1.06±0.011 | 1.11±0.008 |
| Heterozygous (+/-) | 84.83±0.475 | 1.07±0.008 | 1.11±0.005 |
| GNPTAB knockout (-/-) | 76.09±2.483 | 1.04±0.012 | 1.09±0.015 |
| GNPTAB knockout (-/-) + AAV-GNPTAB | 75.10±1.964 | 1.07±0.009 | 1.11±0.017 |

| | | | |
|---|---|---|---|
| Values depicted are mean ± SEM. | | | |

Next, bone mineral density (BMD), bone mineral content (BMC), and body composition were measured. DEXA is an x-ray-based imaging technique for determination of bone mineral and body composition (as body fat mass). AAV-GNPTAB transfer induced relative increases in BMC and BMD in AAV injected KO mice. **FIGS. 10A-10C** shows the raw BMD data obtained before injection (**FIG. 10A**) and relative ratio in BMD (**FIGS. 10B, 10C**) compared before and post injection. These data are also provided in Table 2 below. These results demonstrate a significant effect of the gene transfer on bone mineral density.

**Table 2. Bone mineral density (BMD) change**

| | | Ratio of height increase (Height after indicated interval/starting height) | |
|---|---|---|---|
| Genotype | Starting BMD (g/cm²) | BMD after 16 weeks | BMD after 32 weeks |
| Wild-type (+/+) | 0.055±0.001 | 1.139±0.017 | 1.124±0.032 |
| Heterozygous (+/-) | 0.056±0.001 | 1.137±0.016 | 1.140±0.007 |
| GNPTAB knockout (-/-) | 0.050±0.002 | 1.156±0.011 | 1.157±0.012 |
| GNPTAB knockout (-/-) + AAV-GNPTAB | 0.048±0.001 | 1.219±0.013 | 1.236±0.044 |

| | | | |
|---|---|---|---|
| Values depicted are mean ± SEM. | | | |

Similarly, the raw bone mineral content (BMC) data showed a strong gene therapy effect (**FIG. 11A**). The ratio data (**FIG. 11B, 11C**) also approached a significant effect in bone growth. These data are also provided in Table 3 below.

**Table 3. Bone mineral content (BMC) change**

| | | Ratio of height increase (Height after indicated interval/starting height) | |
|---|---|---|---|
| Genotype | Starting BMD (g/cm²) | BMD after 16 weeks | BMD after 32 weeks |
| Wild-type (+/+) | 0.575±0.016 | 1.172±0.047 | 1.123±0.073 |
| Heterozygous (+/-) | 0.565±0.018 | 1.197±0.045 | 1.116±0.042 |
| GNPTAB knockout (-/-) | 0.448±0.027 | 1.272±0.044 | 1.228±0.049 |
| GNPTAB knockout (-/-) + AAV-GNPTAB | 0.386±0.016 | 1.560±0.080 | 1.551±0.096 |

| | | | |
|---|---|---|---|
| Values depicted are mean ± SEM. | | | |

Next, analysis of body lean content using a DEXA scanner revealed that the lean mass was also reduced in the KO mice (**FIG. 12A**). As shown in **FIG. 12B**, 32 weeks after injection, control mice showed a significant decrease in the percentage of lean mass in comparison to data from before injection. However, no significant changes in lean mass were observed in AAV-GNPTAB treated KO mice. These data are also provided in Table 4 below.

**Table 4. Change in percent lean mass**

| Genotype | Starting % Lean Mass | % Lean Mass after 32 weeks |
|---|---|---|
| Wild-type (+/+) | 86.56±0.627 | 78.98±3.425 |
| Heterozygous (+/-) | 87.44±0.321 | 69.5±3.429 |
| GNPTAB knockout (-/-) | 85.86±0.548 | 70.2±3.313 |
| GNPTAB knockout (-/-) + AAV-GNPTAB | 84.3±0.665 | 84.7±0.7087 |

| | | |
|---|---|---|
| Values depicted are mean ± SEM. | | |

In summary, GNPTAB KO mice failed to thrive and had weak bone density, as is the case in human ML type II. Using this model, the potential of gene therapy using an AAV vector for treatment of ML type II was examined. Overexpressed GNPTAB was found to partially protect bone growth impairment in KO mice. Altogether, the systemic delivery of GNPTAB through AAV vectors was highly efficient and represents a promising approach for the correction of the bone pathology in ML type II.

### SEQUENCES

All polypeptide sequences are presented N-terminal to C-terminal unless otherwise noted. All nucleic sequences are presented 5' to 3' unless otherwise noted.

### GNPTAB

### Human GNPTAB Protein Sequence (SEQ ID NO:1)

### Mouse GNPTAB Protein Sequence (SEQ ID NO:2)

MLLKLLQRQTYTCLSHRYGLYVCFVGVVVTIVSAFQFGEVVLEWSRDQYHVLFDSYRDNIAGKSFQNRLC
LPMPIDVVYTWVNGTDLELLKELQQVREHMEEEQRAMRETLGKNTTEPTKKSEKQLECLLTHCIKVPMLV
LDPPLPANCTLKDLPTLYPSFHAASDMFNVAKPKNPSTNVSVVVFDTTKDVEDAHAGPFKGGSKQMVWRA
YLTTDKEAPGLVLMQGLAFLSGFPPTFKETSQLKTKLPEKLSSKIKLLRLYSEASVALLKLNNPKGFQEL
NKQTKKNMTIDGKELTISPAYLLWDLSAISQSKQDEDVSASRFEDNEELRYSLRSIERHAPWVRNIFIVT
NGQIPSWLNLDNPRVTIVTHQDIFQNLSHLPTFSSPAIESHIHRIEGLSQKFIYLNDDVMFGKDVWPDDF
YSHSKGQKVYLTWPVPNCAEGCPGSWIKDGYCDKACNNSACDWDGGDCSGNTAGNRFVAGGGGTGNIGAG
QHWQFGGGINTISYCNQGCANSWLADKFCDQACNVLSCGFDAGDCGQDHFHELYKVTLLPNQTHYVVPKG
EYLSYFSFANIARRGVEGTYSDNPIIRHASIANKWKTIHLIMHSGMNATTIYFNLTLQNANDEEFKIQIA
VEVDTREAPKLNSTTQKAYESLVSPVTPLPQADVPFEDVPKEKRFPKIRRHDVNATGRFQEEVKIPRVNI
SLLPKEAQVRLSNLDLQLERGDITLKGYNLSKSALLRSFLGNSLDTKIKPQARTDETKGNLEVPQENPSH
RRPHGFAGEHRSERWTAPAETVTVKGRDHALNPPPVLETNARLAQPTLGVTVSKENLSPLIVPPESHLPK
EEESDRAEGNAVPVKELVPGRRLQQNYPGFLPWEKKKYFQDLLDEEESLKTQLAYFTDSKHTGRQLKDTF
ADSLRYVNKILNSKFGFTSRKVPAHMPHMIDRIVMQELQDMFPEEFDKTSFHKVRHSEDMQFAFSYFYYL
MSAVQPLNISQVFHEVDTDQSGVLSDREIRTLATRIHDLPLSLQDLTGLEHMLINCSKMLPANITQLNNI
PPTQEAYYDPNLPPVTKSLVTNCKPVTDKIHKAYKDKNKYRFEIMGEEEIAFKMIRTNVSHVVGQLDDIR
KNPRKFVCLNDNIDHNHKDARTVKAVLRDFYESMFPIPSQFELPREYRNRFLHMHELQEWRAYRDKLKFW
THCVLATLIIFTIFSFFAEQIIALKRKIFPRRRIHKEASPDRIRV

## Claims

1. A recombinant adeno-associated virus (rAAV) particle for use in a method for treating mucolipidosis type II (ML II) or mucolipidosis type III (ML III) in a mammal,
wherein the rAAV particle comprises a rAAV vector, wherein the rAAV vector comprises nucleic acid encoding N-acetylglucosamine-1-phosphate transferase (GNPTAB), at least one AAV inverted terminal repeat (ITR), and a minute virus of mice (MVM) intron,
wherein the GNPTAB is operably linked to a promoter, wherein the promoter is a cytomegalovirus (CMV) enhancer/chicken beta-actin (CBA) promoter,
wherein the CMV enhancer is a shortened enhancer and the CBA promoter is a truncated CBA promoter.

2. The rAAV particle for the use of claim 1, wherein the treating ameliorates one or more symptoms of ML II or ML III, wherein the one or more symptoms of ML II or ML III are skeletal defects, cognitive deficits, delays in the development of gross and fine motor skills, hearing loss, lack of muscle tone, protruded abdomen, umbilical hernias, progressive mucosal thickening of the airways, frequent respiratory infections, thickening and insufficiency of the mitral valve, constipation or diarrhea.

3. The rAAV particle for the use of claim 1, wherein the treating delays to progression of one or more symptoms of ML II or ML III, wherein the one or more symptoms of ML II or ML III are skeletal defects, cognitive deficits, delays in the development of gross and fine motor skills, hearing loss, lack of muscle tone, protruded abdomen, umbilical hernias, progressive mucosal thickening of the airways, frequent respiratory infections, thickening and insufficiency of the mitral valve, constipation or diarrhea.

4. The AAV particle for the use of any one of claims 1-3, wherein
a) the GNPTAB is a human GNPTAB; and/or.
b) the GNPTAB comprises an amino acid sequence that is at least about 80% identical to the amino acid sequence of SEQ ID NO:1; and/or
c) the GNPTAB comprises the amino acid sequence of SEQ ID NO:1.

5. The AAV particle for the use of any one of claims 1-4, wherein the vector comprises:
a) a polyadenylation sequence preferably wherein the polyadenylation sequence is a bovine growth hormone polyadenylation sequence; and/or
b) two ITRs.

6. The AAV particle for the use of any one of claims 1-5, wherein the AAV terminal repeat is an AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV DJ, a goat AAV, bovine AAV, or mouse AAV serotype ITR.

7. The AAV particle for the use of any one of claims 1-6, wherein the AAV particle comprises:
a) an AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV2/2-7m8, AAV DJ, AAV2 N587A, AAV2 E548A, AAV2 N708A, AAV V708K, goat AAV, AAV1/AAV2 chimeric, bovine AAV, mouse AAV, or rAAV2/HBoV1 serotype capsid; and/or.
b) one or more ITRs and capsid derived from the same AAV serotype; or one or more ITRs derived from a different AAV serotype than capsid of the rAAV viral particles, preferably wherein the rAAV particle comprises an AAV8 capsid, and wherein the vector comprises AAV2 ITRs.

8. The AAV particle for the use of any one of claims 1-7, wherein the rAAV particle is produced by transfecting a host cell with nucleic acid encoding the rAAV vector and nucleic acid encoding AAV rep and cap functions, and providing nucleic acid encoding AAV helper functions.

9. The AAV particle for the use of claim 8, wherein the AAV helper functions are provided by transfecting the host cell with nucleic acid encoding the AAV helper functions, preferably, wherein the AAV helper functions are provided by infecting the host cell with an AAV helper virus that provides the AAV helper functions, preferably, wherein the AAV helper virus is an adenovirus, a herpes simplex virus, or a baculovirus.

10. The AAV particle for the use of any one of claims 1-7, wherein the rAAV particle is produced by an AAV producer cell comprising nucleic acid encoding the rAAV vector and nucleic acid encoding AAV rep and cap functions, and providing nucleic acid encoding AAV helper functions.

11. The AAV particle for the use of claim 10, wherein the AAV producer cell comprises nucleic acid encoding AAV helper functions,
preferably, wherein the AAV helper functions are provided by infecting the AAV producer cells with an AAV helper virus that provides the AAV helper functions, preferably, wherein the AAV helper virus is an adenovirus, a herpes simplex virus, or a baculovirus.

12. The AAV particle for the use of any one of claims 1-11, wherein the mammal is a human, preferably wherein the human is a pediatric subject or a young adult.

13. The AAV particle for the use of any one of claims 1-12, wherein the rAAV particle is administered intravenously, intraperitoneally, intra-arterially, intramuscularly, subcutaneously, or intrahepatically, preferably wherein the rAAV particle is administered intravenously.

14. The AAV particle for the use of any one of claims 1-13, wherein the rAAV is administered to more than one location, and/or wherein the administration is repeated.

15. The AAV particle for the use of any one of claims 1-14, wherein the rAAV viral particles are in a pharmaceutical composition, preferably wherein the pharmaceutical composition further comprises a pharmaceutically acceptable carrier.

## Patentansprüche

1. Rekombinantes Partikel von Adeno-assoziiertem Virus (rAAV) zur Verwendung in einem Verfahren zum Behandeln von Mucolipidose Typ II (ML II) oder Mucolipidose Typ III (ML III) bei einem Säugetier,
wobei das rAAV-Partikel einen rAAV-Vektor umfasst, wobei der rAAV-Vektor Nukleinsäure, die für N-Acetylglucosamin-1-Phosphat-Transferase (GNPTAB) kodiert, mindestens eine AAV-invertierte terminale Wiederholung (ITR) und ein Intron von Minutenvirus von Mäusen (MVM) umfasst,
wobei die GNPTAB funktionsfähig mit einem Promotor verbunden ist, wobei der Promotor ein Enhancer von Cytomegalovirus (CMV)/Promoter von Chicken-beta-Actin (CBA) ist,
wobei der CMV-Enhancer ein verkürzter Enhancer und der CBA-Promoter ein abgeschnittener CBA-Promoter ist.

2. rAAV-Partikel zur Verwendung nach Anspruch 1, wobei das Behandeln ein oder mehrere Symptome von ML II oder ML III bessert, wobei das eine oder die mehreren Symptome von ML II oder ML III Skelettdefekte, kognitive Defizite, Verzögerungen in der Entwicklung von Grob- und Feinmotorik, Hörverlust, mangelnder Muskeltonus, vorstehender Bauch, Nabelbrüche, fortschreitende Schleimhautverdickung der Atemwege, häufige Atemwegsinfektionen, Verdickung und Insuffizienz der Mitralklappe, Verstopfung oder Diarrhöe sind.

3. rAAV-Partikel zur Verwendung nach Anspruch 1, wobei das Behandeln ein Fortschreiten eines oder mehrerer Symptome von ML II oder ML III verzögert, wobei das eine oder die mehreren Symptome von ML II oder ML III Skelettdefekte, kognitive Defizite, Verzögerungen in der Entwicklung von Grob- und Feinmotorik, Hörverlust, mangelnder Muskeltonus, vorstehender Bauch, Nabelbrüche, fortschreitende Schleimhautverdickung der Atemwege, häufige Atemwegsinfektionen, Verdickung und Insuffizienz der Mitralklappe, Verstopfung oder Diarrhöe sind.

4. rAAV-Partikel zur Verwendung nach einem der Ansprüche 1-3, wobei:
a) die GNPTAB eine menschliche GNPTAB ist; und/oder
b) die GNPTAB eine Aminosäuresequenz umfasst, die zu mindestens etwa 80 % mit der Aminosäuresequenz von SEQ ID NO: 1 identisch ist; und/oder
c) die GNPTAB die Aminosäuresequenz von SEQ ID NO: 1 umfasst.

5. AAV-Partikel zur Verwendung nach einem der Ansprüche 1-4, wobei der Vektor Folgendes umfasst:
a) eine Polyadenylierungssequenz, vorzugsweise wobei die Polyadenylierungssequenz eine Rinderwachstumshormon-Polyadenylierungssequenz ist; und/oder
b) zwei ITRs.

6. AAV-Partikel zur Verwendung nach einem der Ansprüche 1-5, wobei die terminale AAV-Wiederholung ein AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV DJ, ein Ziegen-AAV, Rinder-AAV oder Maus-AAV-Serotyp ITR ist.

7. AAV-Partikel zur Verwendung nach einem der Ansprüche 1-6, wobei das AAV-Partikel Folgendes umfasst:
a) ein AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV2/2- 7m8, AAV DJ, AAV2 N587A, AAV2 E548A, AAV2 N708A, AAV V708K, Ziegen-AAV, AAV1/AAV2-Chimäre, Rinder-AAV, Maus-AAV oder rAAV2/HBoV1-Serotyp-Kapsid; und/oder.
b) ein oder mehrere ITRs und Capsid, die von demselben AAV-Serotyp abgeleitet sind; oder ein oder mehrere ITRs, die von einem anderen AAV-Serotyp abgeleitet sind als das Capsid der rAAV-Viruspartikel, vorzugsweise wobei das rAAV-Partikel ein AAV8-Capsid umfasst, und wobei der Vektor AAV2-ITRs umfasst.

8. AAV-Partikel zur Verwendung nach einem der Ansprüche 1-7, wobei das rAAV-Partikel durch Transfizieren einer Wirtszelle mit Nukleinsäure, die für den rAAV-Vektor kodiert, und Nukleinsäure, die für AAV-Rep- und -Cap-Funktionen kodiert, und Bereitstellen von Nukleinsäure, die für AAV-Helferfunktionen kodiert, produziert wird.

9. AAV-Partikel zur Verwendung nach Anspruch 8, wobei die AAV-Helferfunktionen durch Transfizieren der Wirtszelle mit Nukleinsäure, die für die AAV-Helferfunktionen kodiert, bereitgestellt werden,
vorzugsweise wobei die AAV-Helferfunktionen durch Infizieren der Wirtszelle mit einem AAV-Helfervirus bereitgestellt werden, das die AAV-Helferfunktionen bereitstellt, vorzugsweise wobei das AAV-Helfervirus ein Adenovirus, ein Herpes-Simplex-Virus oder ein Baculovirus ist.

10. AAV-Partikel für die Verwendung nach einem der Ansprüche 1-7, wobei das rAAV-Partikel von einer AAV-Produktionszelle produziert wird, umfassend Nukleinsäure, die für den rAAV-Vektor kodiert, und Nukleinsäure, die für AAV-Rep- und -Cap-Funktionen kodiert, und Bereitstellen von Nukleinsäure, die für AAV-Helferfunktionen kodiert.

11. AAV-Partikel zur Verwendung nach Anspruch 10, wobei die AAV-Produktionszelle Nukleinsäure umfasst, die für AAV-Helferfunktionen kodiert,
vorzugsweise wobei die AAV-Helferfunktionen durch Infizieren der AAV-Produktionszellen mit einem AAV-Helfervirus bereitgestellt werden, das die AAV-Helferfunktionen bereitstellt, vorzugsweise wobei das AAV-Helfervirus ein Adenovirus, ein Herpes-Simplex-Virus oder ein Baculovirus ist.

12. AAV-Partikel zur Verwendung nach einem der Ansprüche 1-11, wobei das Säugetier ein Mensch ist, vorzugsweise ein pädiatrisches Subjekt oder ein junger Erwachsener.

13. AAV-Partikel zur Verwendung nach einem der Ansprüche 1-12, wobei das rAAV-Partikel intravenös, intraperitoneal, intraarteriell, intramuskulär, subkutan oder intrahepatisch verabreicht wird, vorzugsweise wobei das rAAV-Partikel intravenös verabreicht wird.

14. AAV-Partikel zur Verwendung nach einem der Ansprüche 1-13, wobei das rAAV an mehr als einer Stelle verabreicht wird, und/oder wobei die Verabreichung wiederholt wird.

15. AAV-Partikel zur Verwendung nach einem der Ansprüche 1-14, wobei die rAAV-Viruspartikel in einer pharmazeutischen Zusammensetzung sind, wobei die pharmazeutische Zusammensetzung vorzugsweise ferner einen pharmazeutisch annehmbaren Träger umfasst.

## Revendications

1. Particule de virus adéno-associé recombinant (VAAr) à utiliser dans un procédé de traitement de la mucolipidose de type II (ML II) ou de la mucolipidose de type III (ML III) chez un mammifère,
dans laquelle la particule de VAAr comprend un vecteur de VAAr, dans laquelle le vecteur de VAAr comprend un acide nucléique codant pour la N-acétylglucosamine-1-phosphate transférase (GNPTAB), au moins une répétition terminale inversée (RTI) du VAA, et un intron du virus minute de la souris (MVM),
dans laquelle la GNPTAB est liée de manière opérationnelle à un promoteur, dans laquelle le promoteur est un amplificateur du cytomégalovirus (CMV)/promoteur de la bêta-actine de poulet (CBA),
dans laquelle l'amplificateur du CMV est un amplificateur raccourci et le promoteur de la CBA est un promoteur de la CBA tronqué.

2. Particule de VAAr pour l'utilisation selon la revendication 1, dans laquelle le traitement améliore un ou plusieurs symptômes de la ML II ou ML III, dans laquelle les un ou plusieurs symptômes de la ML II ou ML III sont des défauts squelettiques, des déficits cognitifs, des retards dans le développement de la motricité globale et fine, une perte auditive, un manque de tonus musculaire, un abdomen proéminent, des hernies ombilicales, un épaississement progressif de la muqueuse des voies respiratoires, des infections respiratoires fréquentes, un épaississement et une insuffisance de la valve mitrale, une constipation ou de la diarrhée.

3. Particule de VAAr pour l'utilisation selon la revendication 1, dans laquelle le traitement retarde la progression d'un ou plusieurs symptômes de la ML II ou ML III, dans laquelle les un ou plusieurs symptômes de la ML II ou ML III sont des défauts squelettiques, des déficits cognitifs, des retards dans le développement de la motricité globale et fine, une perte auditive, un manque de tonus musculaire, un abdomen proéminent, des hernies ombilicales, un épaississement progressif de la muqueuse des voies respiratoires, des infections respiratoires fréquentes, un épaississement et une insuffisance de la valve mitrale, une constipation ou de la diarrhée.

4. Particule de VAA pour l'utilisation selon l'une quelconque des revendications 1 à 3, dans laquelle
a) la GNPTAB est une GNPTAB humaine ; et/ou
b) la GNPTAB comprend une séquence d'acides aminés qui est identique à au moins 80 % à la séquence d'acides aminés de SEQ ID N° : 1 ; et/ou
c) la GNPTAB comprend la séquence d'acides aminés de SEQ ID N° : 1.

5. Particule de VAA pour l'utilisation selon l'une quelconque des revendications 1 à 4, dans laquelle le vecteur comprend :
a) une séquence de polyadénylation, de préférence dans laquelle la séquence de polyadénylation est une séquence de polyadénylation de l'hormone de croissance bovine ; et/ou
b) deux RTI.

6. Particule de VAA pour l'utilisation selon l'une quelconque des revendications 1 à 5, dans laquelle la répétition terminale de VAA est une RTl de sérotype AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV DJ, VAA de chèvre, VAA de bovin ou VAA de souris.

7. Particule de VAA pour l'utilisation selon l'une quelconque des revendications 1 à 6, dans laquelle la particule de VAA comprend :
a) une capside de sérotype AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV2/2- 7m8, AAV DJ, AAV2 N587A, AAV2 E548A, AAV2 N708A, AAV V708K, VAA de chèvre, AAV1/AAV2 chimérique, VAA de bovin, VAA de souris ou rAAV2/HBoV1 ; et/ou
b) une ou plusieurs RTl et une capside dérivées du même sérotype de VAA ; ou une ou plusieurs RTl dérivées d'un sérotype de VAA différent de la capside des particules virales de VAAr, de préférence dans laquelle la particule de VAAr comprend une capside AAV8, et dans laquelle le vecteur comprend des RTl AAV2.

8. Particule de VAA pour l'utilisation selon l'une quelconque des revendications 1 à 7, dans laquelle la particule de VAAr est produite par transfection d'une cellule hôte avec l'acide nucléique codant pour le vecteur de VAAr et l'acide nucléique codant pour les fonctions rep et cap du VAA, et fourniture de l'acide nucléique codant pour les fonctions helper du VAA.

9. Particule de VAA pour l'utilisation selon la revendication 8, dans laquelle les fonctions helper du VAA sont fournies en transfectant la cellule hôte avec l'acide nucléique codant pour les fonctions helper du VAA,
de préférence, dans laquelle les fonctions helper du VAA sont fournies en infectant la cellule hôte avec un virus helper du VAA qui fournit les fonctions helper du VAA, de préférence, dans laquelle le virus helper du VAA est un adénovirus, un virus de l'herpès simplex, ou un baculovirus.

10. Particule de VAA pour l'utilisation selon l'une quelconque des revendications 1 à 7, dans laquelle la particule de VAAr est produite par une cellule productrice de VAA comprenant l'acide nucléique codant pour le vecteur de VAAr et l'acide nucléique codant pour les fonctions rep et cap du VAA, et en fournissant l'acide nucléique codant pour les fonctions helper du VAA.

11. Particule de VAA pour l'utilisation selon la revendication 10, dans laquelle la cellule productrice de VAA comprend un acide nucléique codant pour les fonctions helper du VAA,
de préférence, dans laquelle les fonctions helper du VAA sont fournies en infectant la cellule productrice de VAA avec un virus helper du VAA qui fournit les fonctions helper du VAA, de préférence, dans laquelle le virus helper du VAA est un adénovirus, un virus de l'herpès simplex, ou un baculovirus.

12. Particule de VAA pour l'utilisation selon l'une quelconque des revendications 1 à 11, dans laquelle le mammifère est un humain, de préférence un sujet pédiatrique ou un jeune adulte.

13. Particule de VAA pour l'utilisation selon l'une quelconque des revendications 1 à 12, dans laquelle la particule de VAAr est administrée par voie intraveineuse, intrapéritonéale, intra-artérielle, intramusculaire, sous-cutanée ou intrahépatique, de préférence, dans laquelle la particule de VAAr est administrée par voie intraveineuse.

14. Particule de VAA pour l'utilisation selon l'une quelconque des revendications 1 à 13, dans laquelle le VAAr est administré à plus d'un site, et/ou dans laquelle l'administration est répétée.

15. Particule de VAA pour l'utilisation selon l'une quelconque des revendications 1 à 14, dans laquelle les particules virales de VAAr se trouvent dans une composition pharmaceutique, de préférence dans laquelle la composition pharmaceutique comprend en outre un support pharmaceutiquement acceptable.
